(19) Europäisches Patentamt
European Patent Office
Office européen des brevets

(11) Numéro de publication: **0 275 742 B1**

(12) **FASCICULE DE BREVET EUROPEEN**

(45) Date de publication de fascicule du brevet: **03.02.93**

(51) Int. Cl.5: **C07D 263/24**, C07C 217/90

(21) Numéro de dépôt: **87402827.7**

(22) Date de dépôt: **11.12.87**

# (54) Dérivés 5-hydroxyéthylés de l'oxazolidinone-2, leurs procédés de préparation et leurs applications en thérapeutique.

(30) Priorité: **19.12.86 FR 8617845**

(43) Date de publication de la demande:
**27.07.88 Bulletin 88/30**

(45) Mention de la délivrance du brevet:
**03.02.93 Bulletin 93/05**

(84) Etats contractants désignés:
**BE CH DE ES GB GR IT LI LU NL SE**

(56) Documents cités:
**FR-A- 2 458 547**

(73) Titulaire: **DELALANDE S.A.**
**32, rue Henri Regnault**
**F-92400 Courbevoie(FR)**

(72) Inventeur: **Langlois, Michel**
**4, place César Franck**
**F-78530 Buc(FR)**
Inventeur: **Schoofs, Alain-René**
**242, rue de la Croix-Nivert**
**F-75015 Paris(FR)**
Inventeur: **Rumigny, Jean-François**
**17, boulevard Richelieu**
**F-92500 Rueil Malmaison(FR)**

(74) Mandataire: **Kedinger, Jean-Paul et al**
**c/o Cabinet Malemont 42, avenue du Président Wilson**
**F-75116 Paris(FR)**

EP 0 275 742 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouvelles oxazolidinones-2, leurs procédés de préparation et leur application en thérapeutique.

Les oxazolidinones-2 selon l'invention répondent plus précisément à la formule suivante :

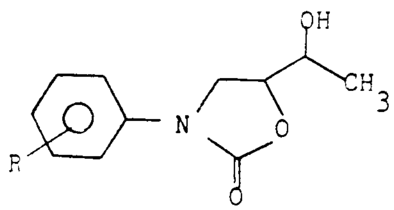

(I)

dans laquelle R représente :

- le groupe benzyloxy ;
- un groupe benzyloxy substitué par un ou deux atomes d'halogène et plus particulièrement un atome de chlore en position méta ; par un groupement alkoxy comportant de 1 à 4 atomes de carbone et situé plus particulièrement en position méta ; ou par un groupement trifluorométhyle situé plus particulièrement en position méta ;
- un radical hydroxy ;
- un groupe alkoxy linéaire ou ramifié, comprenant de 1 à 6 atomes de carbone ; ou
- un groupe cycloalkylalkyloxy comprenant de 4 à 7 atomes de carbone et plus particulièrement un groupe cyclohexylalkoxy.

Du fait que les composés (I) ci-dessus comportent deux atomes de carbone asymétriques dans leur molécule, ils existent chacun sous la forme d'un mélange de 4 stéréoisomères symbolisés, dans ce qui suit, par IA(+), IA(-), IB(+) et IB(-), le symbole A correspondant à la configuration relative érythro et B à la configuration relative thréo. Ce mélange peut être séparé en deux couples de diastéréoisomères racémiques, à savoir le couple érythro IA(+), IA(-) et le couple thréo IB(+), IB(-), pouvant encore être symbolisés par IA(±) et IB(±), chaque couple pouvant lui-même être dédoublé pour isoler les énantiomères correspondants.

La présente invention couvre donc chacun des mélanges de 4 stéréoisomères de formule (I), chacun des couples de diastéréoisomères racémiques correspondants et chacun des énantiomères correspondant à chaque couple, à l'exclusion de 1'(hydroxy-4 phényl)-3 (hydroxy-1 éthyl)-5 oxazolidinone-2 décrit dans FR-A-2 458 547 à titre d'intermédiaire de synthèse.

Conformément à l'invention, les mélanges de 4 stéréoisomères de formule (I) et de structure particulière :

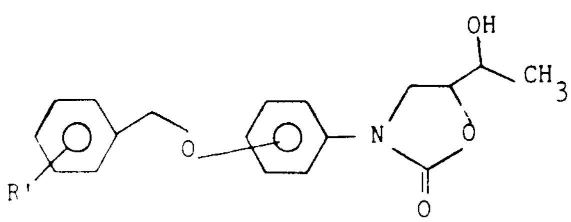

(Ia)

où R' représente un atome d'hydrogène, un ou deux atomes d'halogène, un groupement alkoxy comportant 1 à 4 atomes de carbone ou un groupement trifluorométhyle, sont obtenus par un procédé qui consiste :

- (1) à condenser les anilines de formule :

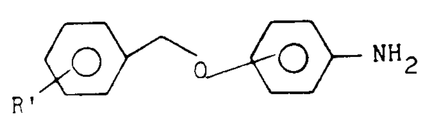

(II)

où R' a la même signification que dans la formule (Ia), respectivement avec le mélange des 4 stéréoisomères de l'époxy-1,2 butanol-3 de formule :

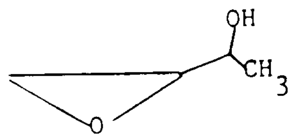

(III)

cette condensation étant de préférence effectuée à chaud dans un solvant organique, en particulier à reflux dans un solvant organique hydroxylé tel que l'isopropanol, puis

- (2) à soumettre chaque mélange résultant de 4 stéréoisomères de formule :

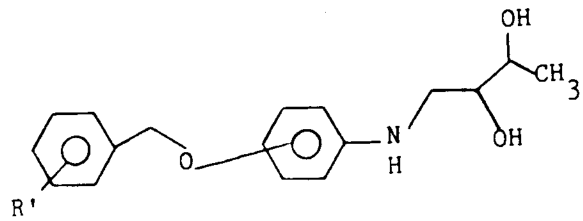

(IV)

à une cyclisation par action du carbonate d'éthyle en présence d'éthylate de sodium, de préférence à chaud dans un solvant organique, en particulier à reflux dans le toluène.

Le mélange des 4 stéréoisomères de formule (III) est quant à lui obtenu par époxydation du butène -3,4 ol-2 de formule :

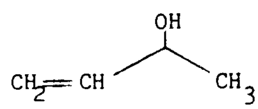

(V)

Pour effectuer cette époxydation, on fait appel à un agent d'époxydation tel que l'acide méta-chloroperbenzoïque et on opère de préférence à température ambiante dans un solvant organique tel que le chlorure de méthylène.

Conformément à la présente invention, chaque mélange de 4 stéréoisomères de formule (Ia) peut être séparé en deux couples de diastéréoisomères racémiques, à savoir un couple (le moins polaire) de configuration relative érythro : IaA(+), IaA(-) [ou IaA(±)] et un couple (le plus polaire) de configuration relative thréo : IaB(+), IaB(-) [ou IaB(±)]. Cette séparation est réalisée par chromatographie sur colonne de silice sous moyenne pression.

Toujours conformément à l'invention, chacun de ces couples peut lui-même être dédoublé pour isoler les énantiomères correspondants. Le dédoublement du couple érythro IaA(±) peut par exemple être réalisé par estérification à l'aide du chlorure de l'acide phénoxy-2 propionique (+) (VI) dans la pyridine, suivant le schéma suivant :

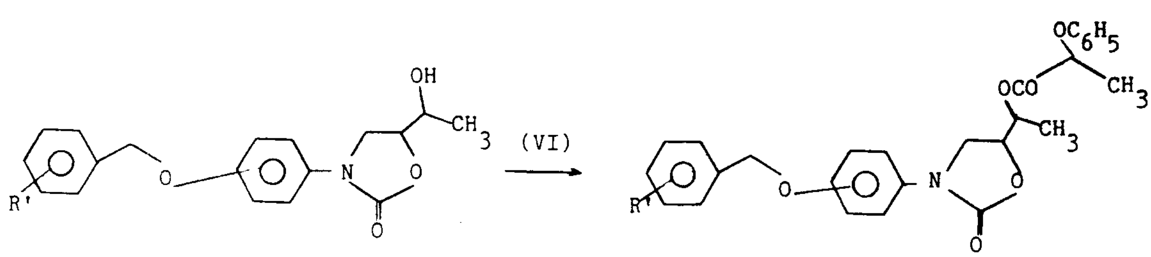


IaA(±) I'aA(±)

Cette estérification est suivie d'une séparation par chromatographie sur colonne de silice sous moyenne pression du couple racémique de diastéréoisomères érythro I'aA(±) pour donner d'une part l'énantiomère

ester le moins polaire I'aA(+) et d'autre part l'énantiomère ester le plus polaire I'aA(-).

Les deux énantiomères ester I'aA(+) et I'aA(-) ainsi isolés sont ensuite saponifiés, par exemple à la soude, pour obtenir respectivement l'énantiomère IaA(+) et l'énantiomère IaA(-).

Quant au dédoublement du couple thréo IaB(±), il peut être réalisé par estérification à l'aide du chlorure de l'acide α-méthoxy α-trifluorométhylphényl acétique (-) (VII) dans la pyridine suivant le schéma suivant :

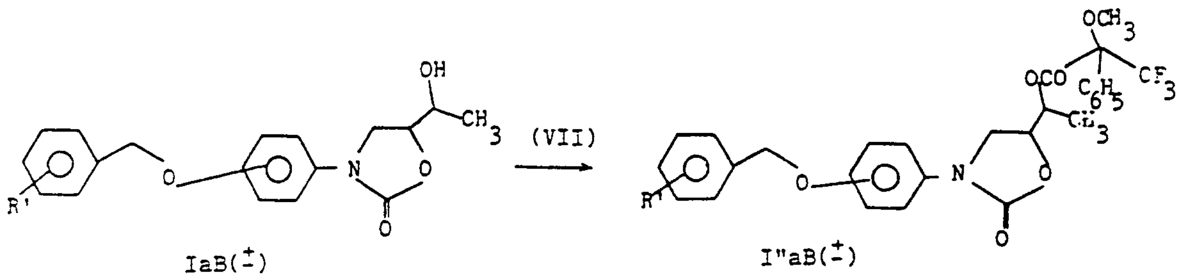


IaB(±) I"aB(±)

Cette estérification est suivie d'une séparation par chromatographie sur colonne de silice sous moyenne pression du couple racémique de diastéréoisomères thréo I"aB(±) pour donner d'une part l'énantiomère ester le moins polaire I"aB(+) et d'autre part l'énantiomère ester le plus polaire I"aB(-).

Les deux énantiomères ester ainsi isolés sont ensuite saponifiés, par exemple à la soude, pour obtenir respectivement l'énantiomère IaB(+)et l'énantiomère IaB(-).

Il convient de préciser par ailleurs, que la configuration absolue de chacun des énantiomères IaA(+), IaA(-), IaB(+) et IaB(-) a été déterminée par la méthode du dédoublement cinétique partiel de l'anhydride α-phénylbutyrique par ces énantiomères (Weidmann R., Schoofs A.R. et Horeau A., Comptes-Rendus Acad. Sci., Paris, Série II, 1984, p. 319 et références citées). Ainsi :

- à l'énantiomère IaA(+) a pu être attribuée la configuration absolue (S, R),
- à l'énantiomère IaA(-) a pu être attribuée la configuration absolue (R, S),
- à l'énantiomère IaB(+) a pu être attribuée la configuration absolue (S, S), et
- à l'énantiomère IaB(-) a pu être attribuée la configuration absolue (R, R).

La débenzylation respective des énantiomères IaA(+), IaA(-), IaB(+), IaB(-) conduit aux énantiomères de formule (I) et de structure particulière :

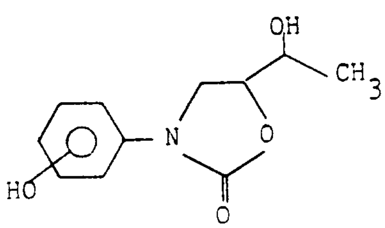

(Ib)

c'est-à-dire aux énantiomères IbA(+), IbA(-), IbB(+) et IbB(-).

Cette débenzylation peut notamment être effectuée par hydrogénolyse en présence d'un catalyseur d'hydrogénolyse tel que le palladium sur charbon dans un solvant organique, de préférence un solvant organique hydroxylé tel que l'éthanol.

L'alkylation sélective du radical hydroxyle phénolique de chacun des énantiomères IbA(+), IbA(-), IbB(+) et IbB(-), au moyen d'un agent d'alkylation de formule :

R" - X (VIII)

où R" représente :

- un groupe alkyle linéaire ou ramifié, comprenant de 1 à 6 atomes de carbone, ou
- un groupe cycloalkylalkyle comprenant de 4 à 7 atomes de carbone,

et X représente un groupe bon partant tel qu'un atome de chlore ou de brome, conduit à l'obtention des énantiomères de formule(I) et de structure particulière :

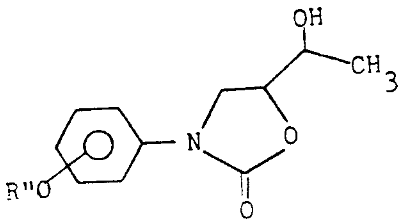

(Ic)

où R" a la même signification que dans la formule (VIII), c'est-à-dire aux énantiomères IcA(+), IcA(-), IcB-(+) et IcB(-).

Cette alkylation sélective est de préférence réalisée dans un solvant organique, en particulier un solvant polaire tel que l'acétonitrile, le DMF ou la butanone-2, en présence d'une base minérale telle que le carbonate de potassium.

Les préparations suivantes sont données à titre d'exemples pour illustrer l'invention.

Exemple 1 : Synthèse du mélange des 4 stéréoisomères de l'époxy-1,2 butanol-3 [III] (numéro de code 790255)

A une solution refroidie à 0°C de 83,6 g (O,48 mole) d'acide métachloroperbenzoïque dans 25O ml de $CH_2Cl_2$ est ajoutée goutte à goutte une solution de 33,3 g (0,46 mole) de butène-3,4 ol-2 dans 100 ml de $CH_2Cl_2$. Après 2O heures d'agitation à 2O° C, le milieu partiellement gélifié est refroidi à -18°C puis filtré. La phase organique est brassée pendant 3O' en présence de 10 g de $Na_2CO_3$ solide. Après filtration et évaporation sous très léger vide, on récupère 36 g d'une huile jaune clair (rendement : 88 %) correspondant au mélange attendu et composée de 65 % d'isomère thréo et 35 % d'isomère érythro.

Exemple 2 : Synthèse du mélange des 4 stéréoisomères du [(chloro-3 benzyloxy)-4 phénylaminol-1 butane diol-2,3 [IV] (numéro de code 340245)

Une solution contenant 5O g (O,21 mole) de 4-(chloro-3'-benzyloxy) aniline et 21,6 g (O,24 mole) du mélange obtenu à l'exemple 1, dans 85O ml d'isopropanol est portée à reflux pendant 6 h 30. Après évaporation de l'isopropanol, le mélange brut est purifié par chromatographie flash sur silice (éluant : $CH_2Cl_2$-$CH_3OH$ : 97-3). On récupère 34 g (rendement : 49 %) de solide blanc correspondant au produit attendu et ayant un point de fusion de 95-96° C.

Exemple 3 : Préparation du mélange des 4 stéréoisomères de la [(chloro-3 benzyloxy)-4 phényl]-3 (hydroxy-1 éthyl)-5 oxazolidinone-2 [Ia]

A partir d'une solution de 6OO ml de toluène contenant 33,4 g (O,10 mole) du mélange obtenu à l'exemple 2 sont distillés 150 ml de solvant sous atmosphère d'argon. On ajoute ensuite successivement 15 g (15,5 ml ; 0,127 mole) de carbone d'éthyle puis 3,4 ml d'une solution de EtONa dans EtOH anhydre 1M. Après 4 h 3O à reflux, la réaction de cyclisation est complète. Le milieu réactionnel est concentré puis repris par 300ml de méthyl éthyl cétone. Après lavage avec HCl 2N, puis avec une solution saturée de NaCl, on récupère 35 g de solide brun correspondant au produit attendu.

Exemple 4 : Séparation des deux couples de diastéréoisomères racémiques [IaA(±) et IaB(±)] du [(chloro-3 benzyloxy)-4 phényl]-3 (hydroxy-1 éthyl)-5 oxazolidinone-2 (Ia).

On soumet à une chromatographie sous moyenne pression (éluant : acétate d'éthyle - heptane : 70-30), le mélange obtenu à l'exemple 3 et on isole ainsi 2 produits à mobilité différente sur plaque de $SiO_2$ :

- le produit le plus mobile (le moins polaire) - 11,4 g d'un solide blanc ayant un point de fusion de 88° C - étant constitué par le couple de diastéréoisomères racémique de configuration relative érythro [IaA(±) ; numéro de code : 300868], et
- le produit le moins mobile (le plus polaire) - 18,7 g d'un solide blanc ayant un point de fusion de 114,5° C - étant constitué par le couple de diastéréoisomères racémique de configuration relative thréo (IaB(±) ; numéro de code 300869].

Exemple 5 : Dédoublement du couple de diastéréoisomères racémique thréo [IaB(±) ; numéro de code : 300869] du [(chloro-3 benzyloxy)-4 phényl]-3 (hydroxy-1 éthyl)-5 oxazolidinone-2 [Ia].

Dans un ballon sous argon contenant 9 g (0,035 mole) du chlorure de l'acide α-méthoxy α-trifluorométhylphénylacétique ($[\alpha]_D^{20}$ = - 134°6 (C=5,2 ; $CCl_4$)) et 30 ml de pyridine, sont additionnés à 0° C en plusieurs fois 11,2 g (0,032 mole) du couple IaB(±) obtenu à l'exemple 4. Après solubilisation à l'aide de 25 ml de pyridine, 1,2 g (0,014 mole) de 4-diméthylaminopyridine sont ajoutés. Au bout de 60 heures d'agitation à température ambiante, la réaction est complète. On verse sur 50 ml d'eau, puis extrait 3 fois à l'éther. La phase organique est lavée 3 fois avec HCl 2N, puis par une solution saturée de $NaHCO_3$, séchée sur $MgSO_4$ et évaporée.

Le mélange brut obtenu est chromatographié sur colonne de silice sous moyenne pression (éluant = acétate d'éthyle-hexane : 50-50) pour donner les deux esters de MOSHER séparés I''aB(+) et I''aB(-) sous forme d'huile :

- énantiomère ester I''aB(+) (le plus mobile sur plaque $SiO_2$) : 7,9 g ; $(\alpha)_D^{20}$ = + 25,3° (C = 1,0 ; $CH_2Cl_2$)
- énantiomère ester I''aB(-) (le moins mobile sur plaque $SiO_2$) : 7,6 g ; $(\alpha)_D^{20}$ = - 73,7° (C = 1,1 ; $CH_2Cl_2$)

A une solution refroidie à -10° C de 3,6 g (0,0064 mole) de l'énantiomère ester I''aB(+) dans 50 ml de $CH_3OH$ sont additionnés 4,8 ml de NaOH 2N. La saponification est complète après 8 heures d'agitation à 20° C.

Après concentration à froid, le milieu réactionnel est étendu par 160 ml de méthyl éthyl cétone et 50 ml de mélange glace-eau. Après décantation, la phase organique est lavée par une solution saturée de NaCl, séchée sur $MgSO_4$, puis évaporée. Par chromatographie flash (éluant = acétate d'éthyle - hexane : 70-30), on récupère 2 g (rendement = 90 %) d'un solide blanc (F = 90°C) qui est l'énantiomère thréo (+) [IaB(+) ; numéro de code : 300872] du [(chloro-3 benzyloxy)-4 phényl]-3 (hydroxy-1 éthyl)-5 oxazolidinone-2 :

. $[\alpha]_D^{20}$ = + 39,5° (C = 1,03 ; $CH_2Cl_2$)
. configuration absolue : (S,S).

La saponification dans les mêmes conditions que ci-dessus, de l'énantiomère ester I''aB(-), conduit à l'énantiomère thréo (-) [IaB(-) ; numéro de code : 300873] du [(chloro-3 benzyloxy)-4 phényl]-3 (hydroxy-1 éthyl)-5 oxazolidinone-2 :

. point de fusion : 90°C
. $[\alpha]_D^{20}$ = - 41,5° (C = 1,01 ; $CH_2Cl_2$)
. Configuration absolue : (R,R).

Exemple 6 : Dédoublement du couple de diastéréoisomers racémique érythro [IaA(±); numéro de code : 300868] du [(chloro-3 benzyloxy)-4 phényl]-3 (hydroxy-1 éthyl)-5 oxazolidinone-2 [Ia].

A une solution refroidie à 0°C de 4,25 g (0,023 mole) du chlorure de l'acide phénoxy-2 propionique {-$[\alpha]_D^{20}$ = + 26,3° (C = 1,0 ; $CH_2Cl_2$)} et 0,65 g (0,00053 mole) de 4-diméthylaminopyridine dans 50 ml de pyridine, sont additionnés en 30' 6,15 g du couple IaA(±) obtenu à l'exemple 4, dissous dans 30 ml de pyridine. La réaction d'estérification est complète après 16 heures d'agitation à 20°C. Le milieu réactionnel est traité comme lors de l'estérification de l'exemple 5. Par chromatographie moyenne pression (éluant = acétate d'éthyle - hexane : 40-60), on récupère :

- l'énantiomère ester I'aA(+) [le plus mobile sur plaque de $SiO_2$] : 3,51 g ; $[\alpha]_D^{20}$ = +37,6° (C = 1,0 ; $CH_2Cl_2$) ; et
- l'énantiomère ester I'aA(-) [le moins mobile sur plaque de $SiO_2$] : 3,66 g ; $[\alpha]_D^{20}$ = - 14,2° (C = 1,0 ; $CH_2Cl_2$).

La saponification par NaOH 2N (1,3 éq.) de ces énantiomères ester pendant 2 heures à 20° C dans les mêmes conditions opératoires que dans l'exemple 5, suivie d'une extraction classique à l'acétate d'éthyle permet d'isoler respectivement :

- l'énantiomère érythro (+) [IaA(+) ; numéro de code : 340177] du [(chloro-3 benzyloxy)-4 phényl]-3 (hydroxy-1 éthyl)-5 oxazolidinone-2 :
  . Point de fusion : 78°C
  . $[\alpha]_D^{20}$ = + 16,6° (C = 1,0 ; $CH_2Cl_2$)
  . Configuration absolue : (S,R) ; et
- l'énantiomère érythro (-) [IaA(-) ; numéro de code : 340176] du [(chloro-3 benzyloxy)-4 phényl]-3 (hydroxy-1 éthyl)-5 oxazolidinone-2 :
  . Point de fusion : 78°C

. $[\alpha]_D$ = - 16,2° (C = 1,0 ; $CH_2Cl_2$)

. Configuration absolue : (R,S).

Par mise en oeuvre des procédés objet des exemples 1 à 6 ci-dessus, mais en partant des réactifs appropriés, on obtient les autres composés (Ia) dont certains sont répertoriés dans le tableau I ci-après.

Exemple 7 : Préparation de l'énantiomère thréo (-) (R,R) de l'[hydroxy-4 phényl]-3 (hydroxy-1 éthyl)-5 oxazolidinone-2 [IbB(-) ; numéro de code : 340 315]

A une fiole de 1 litre contenant 4 g de Pd/C à 10 % en suspension dans 350 ml d'éthanol à 95 % sont introduits 39,3 g (0,125 mole) de l'énantiomère de numéro de code 300873 préparé à l'exemple 5. Après 2 heures d'agitation forte sous atmosphère d'hydrogène, la réaction d'hydrogénolyse est complète. Le catalyseur est récupéré par filtration et le filtrat est évaporé sous pression réduite pour laisser une huile jaune clair qui cristallise. Un brassage d'une heure dans le pentane donne 27 g (rendement : 97 %) d'un solide blanc correspondant au produit attendu :

. Point de fusion : 149° C

. $[\alpha]_D^{20}$ = -73,2 ° (C = 1,0 ; $CH_3OH$)

Les autres énantiomères de l'[hydroxy-4 phényl]-3 (hydroxy-1 éthyl)-5 oxazolidinone-2 sont obtenus par un processus analogue à partir des réactifs appropriés et sont rassemblés dans le tableau II ci-après.

Exemple 8 : Préparation de l'énantiomère thréo (-) (R,R) de l'[(éthyl-2 butyloxy)-4 phényl]-3 (hydroxy-1 éthyl)-5 oxazolidinone-2 [IcB(-) ; numéro de code : 200520].

Dans un bicol de 25 ml surmonté d'un réfrigérant sont introduits 1 g (4,5 mmoles) de l'énantiomère préparé à l'exemple 7, 1,5 g (9 mmoles) de bromo-1 éthyl-2 butane et 1,85 g (14 mmoles) de $K_2CO_3$ dans 10 ml de DMSO. Après 16 heures d'agitation à 60° C l'alkylation est complète. Le milieu réactionnel est versé sur un mélange eau-glace de 150 ml. Après 3 extractions par 100 ml d'éther, les phases organiques réunies sont lavées par une solution saturée de NaCl, séchées sur $MgSO_4$, filtrées et évaporées. On recueille après flash chromatographie (éluant = $CH_2Cl_2$ - $CH_3OH$ : 98-2), 0,9 g (rendement : 66 %) d'un solide incolore correspondant au produit attendu :

. Point de fusion : 63° C

. $[\alpha]_D^{20}$ = - 46,2° (C = 1 ; $CH_2Cl_2$)

Par mise en oeuvre du procédé objet de l'exemple 8, mais en partant des réactifs appropriés, on obtient les autres composés (Ic) dont certains sont répertoriés dans le tableau III ci-après.

TABLEAU I

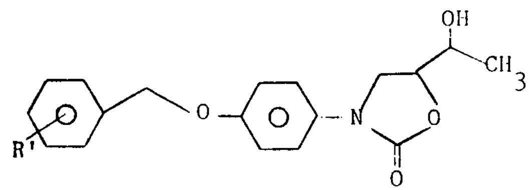

(Ia)

| Numéro de Code | R' | Configuration absolue ou relative | $[\alpha]_D^{20}$ (C = 1 ; $CH_2Cl_2$) | Formule brute | Poids moléculaire | Point de fusion (°C) | ANALYSE ELEMENTAIRE % | C | H | N |
|---|---|---|---|---|---|---|---|---|---|---|
| 300873 | 3-Cl | (R, R) | - 41,5° | $C_{18}H_{18}ClNO_4$ | 347,789 | 90 | Cal. | 62,16 | 5,22 | 4,03 |
| | | | | | | | Tr. | 61,99 | 5,23 | 3,76 |
| 300872 | 3-Cl | (S, S) | + 39,5° | $C_{18}H_{18}ClNO_4$ | 347,789 | 90 | Cal. | 62,16 | 5,22 | 4,03 |
| | | | | | | | Tr. | 61,99 | 5,28 | 3,88 |
| 340176 | 3-Cl | (R, S) | - 16,2° | $C_{18}H_{18}ClNO_4$ | 347,789 | 78 | Cal. | 62,16 | 5,22 | 4,03 |
| | | | | | | | Tr. | 61,85 | 5,41 | 3,78 |
| 340177 | 3-Cl | (S, R) | + 16,6° | $C_{18}H_{18}ClNO_4$ | 347,789 | 80 | Cal. | 62,16 | 5,22 | 4,03 |
| | | | | | | | Tr. | 62,77 | 5,48 | 4,09 |

TABLEAU I (suite)

| Numéro de Code | R' | Configuration absolue ou relative | $[\alpha]_D^{20}$ (C = 1 ; $CH_2Cl_2$) | Formule brute | Poids moléculaire | Point de fusion (°C) | ANALYSE ELEMENTAIRE % | C | H | N |
|---|---|---|---|---|---|---|---|---|---|---|
| 300868 | 3-Cl | érythro | (±) | $C_{18}H_{18}ClNO_4$ | 347,789 | 88 | Cal. | 62,16 | 5,22 | 4,03 |
| | | | | | | | Tr. | 61,86 | 5,47 | 3,95 |
| 300869 | 3-Cl | thréo | (±) | $C_{18}H_{18}ClNO_4$ | 347,789 | 114,5 | Cal. | 62,16 | 5,22 | 4,03 |
| | | | | | | | Tr. | 61,97 | 5,23 | 4,28 |
| 200226 | H | (R, R) | - 46,9° | $C_{18}H_{19}NO_4$ | 313,340 | 126 | Cal. | 68,99 | 6,11 | 4,47 |
| | | | | | | | Tr. | 69,30 | 6,36 | 4,50 |
| 200435 | 3-F | (R, R) | - 44,3° | $C_{18}H_{18}FNO_4$ | 331,332 | 108 | Cal. | 65,25 | 5,48 | 4,23 |
| | | | | | | | Tr. | 65,47 | 5,51 | 4,35 |

TABLEAU I (suite)

| Numéro de Code | R' | Configuration absolue ou relative | $[\alpha]_D^{20}$ (C = 1 ; $CH_2Cl_2$) | Formule brute | Poids moléculaire | Point de fusion (°C) | ANALYSE ELEMENTAIRE | | | |
|---|---|---|---|---|---|---|---|---|---|---|
| | | | | | | | % | C | H | N |
| 200441 | 4-F | (R, R) | - 44,0° | $C_{18}H_{18}FNO_4$ | 331,332 | 125 | Cal. | 65,25 | 5,48 | 4,23 |
| | | | | | | | Tr. | 65,35 | 5,55 | 4,39 |
| 200498 | 3-Cl, 4-F | (R, R) | - 40,4° | $C_{18}H_{17}ClFNO_4$ | 365,781 | 97 | Cal. | 59,10 | 4,68 | 3,83 |
| | | | | | | | Tr. | 58,84 | 4,62 | 3,54 |
| 200436 | 3-$OCH_3$ | (R, R) | - 41,9° | $C_{19}H_{21}NO_5$ | 343,366 | 116 | Cal. | 66,46 | 6,16 | 4,08 |
| | | | | | | | Tr. | 66,71 | 6,43 | 4,30 |

TABLEAU I (suite)

| Numéro de Code | R' | Configuration absolue ou relative | $[\alpha]_D^{20}$ (C = 1 ; $CH_2Cl$) | Formule brute | Poids moléculaire | Point de fusion (°C) | ANALYSE ELEMENTAIRE | | | |
|---|---|---|---|---|---|---|---|---|---|---|
| | | | | | | | % | C | H | N |
| 200434 | 3-$CF_3$ | (R, R) | - 38,9° | $C_{19}H_{18}F_3NO_4$ | 381,342 | 92 | Cal. | 59,84 | 4,76 | 3,67 |
| | | | | | | | Tr. | 60,00 | 4,78 | 3,97 |
| | | | | | | | | | | |
| | | | | | | | | | | |
| | | | | | | | | | | |
| | | | | | | | | | | |
| | | | | | | | | | | |
| | | | | | | | | | | |

TABLEAU II

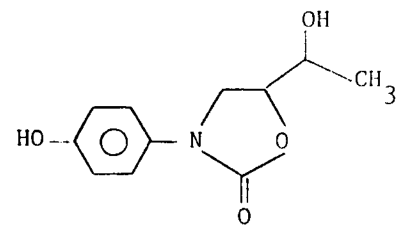

(Ib)

| Numéro de Code | Configuration absolue | $[\alpha]_D^{20}$ (C = 1 ; $CH_3OH$) | Formule brute | Poids moléculaire | Point de fusion (°C) | ANALYSE ELEMENTAIRE % | C | H | N |
|---|---|---|---|---|---|---|---|---|---|
| 340 315 | (R,R) | - 73,2° | $C_{11}H_{13}NO_4$ | 223,222 | 149 | Cal. | 59,18 | 5,87 | 6,28 |
| | | | | | | Tr. | 59,42 | 6,11 | 6,13 |
| 200158 | (S, S) | + 71,7° | $C_{11}H_{13}NO_4$ | 223,222 | 154 | Cal. | 59,18 | 5,87 | 6,28 |
| | | | | | | Tr. | 58,91 | 5,79 | 6,13 |
| 200356 | (R, S) | - 28,5° | $C_{11}H_{13}NO_4$ | 223,222 | 163 | Cal. | 59,18 | 5,87 | 6,28 |
| | | | | | | Tr. | 58,88 | 5,73 | 6,12 |
| 200357 | (S, R) | + 29,8° | $C_{11}H_{13}NO_4$ | 223,222 | 163 | Cal. | 59,18 | 5,87 | 6,28 |
| | | | | | | Tr. | 59,14 | 5,85 | 6,09 |

TABLEAU III

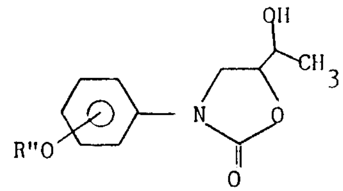

(Ic)

| Numéro de Code | R"O | Configuration absoluc | $[\alpha]_D^{20}$ (C = 1 ; $CH_2Cl_2$) | Formule brute | Poids moléculaire | Point de fusion (°C) | ANALYSE ELEMENTAIRE | | | |
|---|---|---|---|---|---|---|---|---|---|---|
| | | | | | | | % | C | H | N |
| 200225 | 4-$OCH_3$ | (R, R) | - 57,1° | $C_{12}H_{15}NO_4$ | 237,248 | 115 | Cal. | 60,75 | 6,37 | 5,90 |
| | | | | | | | Tr. | 60,87 | 6,60 | 5,97 |
| 200471 | 4-$OC_4H_9$ | (R, R) | - 48,3° | $C_{15}H_{21}NO_4$ | 279,326 | 115 | Cal. | 64,49 | 7,58 | 5,01 |
| | | | | | | | Tr. | 64,26 | 7,63 | 5,01 |
| 200497 | 4-$OiC_5H_{11}$ | (R, R) | - 46,4° | $C_{16}H_{23}NO_4$ | 293,352 | 100 | Cal. | 65,50 | 7,90 | 4,78 |
| | | | | | | | Tr. | 65,35 | 7,89 | 4,58 |
| 200520 | 4-O 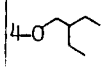 | (R, R) | - 46,2° | $C_{17}H_{25}NO_4$ | 307,378 | 63 | Cal. | 66,42 | 8,20 | 4,56 |
| | | | | | | | Tr. | 66,27 | 8,33 | 4,60 |

TABLEAU III (suite)

| Numéro de Code | R"O | Configuration absolue | $[\alpha]_D^{20}$ (C = 1 ; $CH_2Cl_2$) | Formule brute | Poids moléculaire | Point de fusion (°C) | ANALYSE ELEMENTAIRE % | C | H | N |
|---|---|---|---|---|---|---|---|---|---|---|
| 200443 | 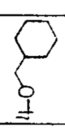 | (R, R) | - 44,2° | $C_{18}H_{25}NO_4$ | 319,388 | 146 | Cal. | 67,69 | 7,89 | 4,39 |
| | | | | | | | Tr. | 67,74 | 7,95 | 4,29 |

Les composés selon l'invention ont été testés sur l'animal de laboratoire et ont fait preuve d'une activité pharmacologique et notamment une activité inhibitrice de la monoamine oxydase, en particulier de la monoamine oxydase de type B.

Cette activité a été mise en évidence par mise en oeuvre du protocole décrit par DOSTERT P. et coll. dans J. Pharm. Pharmacol., 1983, 35, 161-165 ; ce protocole est basé sur des tests enzymatiques menés avec ou sans préincubation notamment à 37° C pendant 20 minutes et il permet de mesurer in vitro l'effet

inbibiteur à l'égard de la MAO-A et de la MAO-B du cerveau de rat.

Le tableau IV ci-après rassemble les constantes d'inhibition Ki(A) et Ki(B) respectivement des formes A et B de la MAO, obtenues pour un certain nombre de composés selon l'invention en suivant le protocole ci-dessus appliqué à un homogénat de cerveau total de rat mâle Sprague-Dawley, à 1 g de tissu/16 ml de tampon phosphate de pH 7,4.

TABLEAU IV

| Composé testé (Numéro de code) | Ki (A)* en nM | Ki (B)* en nM | Ki (A) / Ki (B) |
|---|---|---|---|
| 300873 | > 17240 | 27 | > 640 |
| 200443 | 10344 | 40 | 259 |
| 200497 | 6000 | 168 | 36 |
| 200498 | 9000 | 14 | 634 |
| 300872 | > 1000 | 1600 | > 0,6 |
| 340176 | 476 | 100 | 5 |
| 340177 | 2259 | 343 | 7 |

* Déterminées avec préincubation de 20 mn et calculées d'après la formule :

Ki = CI 50/1 + [S]/$K_M$ avec :

[S] = 480 µM pour la [$^{14}$C] sérotonine, $K_M$ = 100 µM

[S] = 12 µM pour la [$^{14}$C] phényléthylamine, $K_M$ = 6 µM

substrats sélectifs des formes A et B de la MAO.

Il ressort de ce tableau IV que les composés selon l'invention présentent une activité inhibitrice de la MAO et en particulier que les composés de configuration absolue (R,R) sont des inhibiteurs très sélectifs de la MAO de type B.

Par ailleurs, une étude toxicologique sub-aigüe 15 jours effectuée sur le rat a révélé l'inocuité des composés selon l'invention à des doses aussi élevées que 1000 mg/kg/p.o.

Il découle de ce qui précède que les composés de l'invention trouvent leur application en thérapeutique, notamment en tant que médicaments inhibiteurs de la monoamine oxydase, en particulier de la monoamine oxydase de type B. Ils pourront donc être utilisés pour le traitement des dépressions, de la maladie de Parkinson et des déficits neurologiques notamment liés à la sénescence.

L'invention s'étend aux compositions pharmaceutiques renfermant, à titre de principe actif, au moins un composé selon l'invention en association avec un véhicule pharmaceutiquement acceptable. Ces compositions seront administrées par voie orale sous forme de comprimés, dragées ou gélules par exemple, à des doses de principe actif pouvant aller jusqu'à 50 mg/jour en une ou plusieurs prises, par voie rectale sous forme de suppositoires contenant jusqu'à 300 mg de principe actif (1 à 2 par jour) ou encore sous forme de solutés injectables contenant jusqu'a 300 mg de principe actif (1 à 2 injections par jour).

**Revendications**

**Revendications pour les Etats contractants suivants : BE, CH, DE, GB, IT, LI, LU, NL, SE**

**1.** Mélange des 4 stéréoisomères de formule (I) ci-dessous, chacun des couples de diastéréoisomères

racémiques correspondants et chacun des énantiomères correspondant à chaque couple :

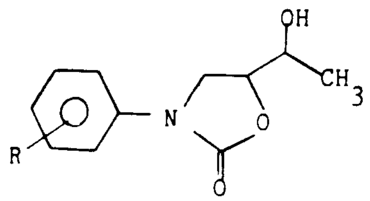

(I)

dans laquelle R représente :

- le groupe benzyloxy ;
- le groupe benzyloxy substitué par un ou deux atomes d'halogène ; par un groupement alkoxy comportant de 1 à 4 atomes de carbone ; ou par un groupement trifluoromethyle ;
- un radical hydroxy ;
- un groupe alkoxy linéaire ou ramifié, comprenant de 1 à 6 atomes de carbone ; ou
- un groupe cycloalkylalkyloxy comprenant de 4 à 7 atomes de carbone, à l'exclusion de l'-(hydroxy-4 phényl)-3 (hydroxy-1 éthyl)-5 oxazolidinone-2.

**2.** Mélange, couples et énantiomères selon la revendication 1 pour lesquels R est en position para et représente le groupe benzyloxy ou un groupe benzyloxy substitué par 3-F ; 3-Cl ; 4-F ; 3-F, 4-Cl ; 3-$OCH_3$ ; ou 3-$CF_3$.

**3.** Mélange, couples et énantiomères selon la revendication 1, pour lesquels R est en position para et représente le groupe méthoxy, butoxy, i-pentoxy, éthyl-2 butoxy ou cyclohexylméthyloxy.

**4.** Enantiomères selon l'une des revendications 1 à 3, de configuration relative thréo.

**5.** Enantiomères selon la revendication 4, de configuration absolue (R, R).

**6.** Médicament possédant notamment une activité inhibitrice de la monoamine oxydase en particulier de type B, caractérisé en ce qu'il est constitué par le mélange, un couple ou un énantiomère selon l'une des revendications 1 à 5.

**7.** Composition pharmaceutique possédant notamment une activité inhibitrice de la monoamine oxydase en particulier de type B, caractérisée en ce qu'elle comprend le mélange, un couple ou un énantiomère selon l'une des revendications 1 à 5, en association avec un véhicule pharmaceutiquement acceptable.

**8.** Procédé de préparation du mélange de 4 stéréoisomères de formule (I) selon la revendication 1 et de structure particulière :

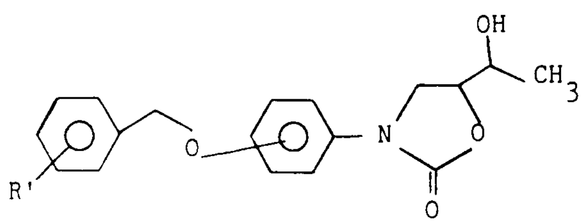

(Ia)

où R' représente un atome d'hydrogène, un ou deux atomes d'halogène ou un groupement alkoxy de 1 à 4 atomes de carbone ou trifluorométhyle, caractérisé en ce qu'il consiste à soumettre le mélange de 4 stéréoisomères de formule :

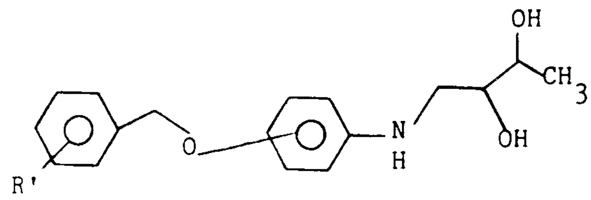

(IV)

où R' a la même signification que dans la formule (Ia), à une cyclisation par action du carbonate d'éthyle en présence d'éthylate de sodium.

**9.** Procédé de préparation du couple racémique de diastéréoisomères érythro et du couple racémique de diastéréoisomères thréo, correspondant à la formule (I) selon la revendication 1 et à la structure particulière (Ia) définie à la revendication 8, caractérisé en ce qu'il consiste à soumettre le mélange des 4 stéréoisomères de formule (I) et de structure particulière (Ia) définie à la revendication 8, à une chromatographie sur colonne de silice sous moyenne pression, ce qui permet d'isoler deux produits, le produit le moins polaire correspondant au couple racémique de diastéréoisomères érythro [IaA(±)] et le produit le plus polaire correspondant au couple racémique de diastéréoisomères thréo [IaB(±)].

**10.** Procédé de préparation de l'énantiomère érythro (+) [IaA(+)] et de l'énantiomère érythro(-)[IaA(-)], correspondant à la formule (I) selon la revendication 1 et à la structure particulière (Ia) définie à la revendication 8, caractérisé en ce qu'il consiste :

(1) à séparer par chromatographie sur colonne de silice sous moyenne pression le couple racémique de diastéréoisomères érythro correspondant à la structure :

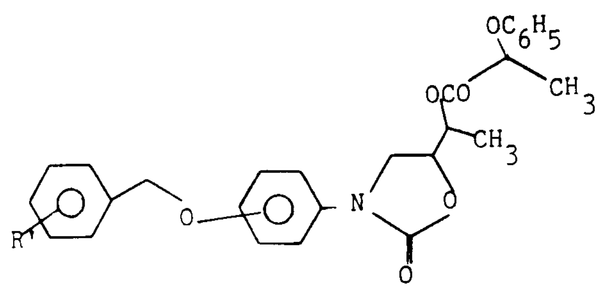

I'aA(±)

où R' a la même signification que dans la revendication 8, ce qui conduit à deux produits, le produit le moins polaire étant constitué par l'énantiomère ester I'aA(+) et le plus polaire par l'énantiomère ester I'aA(-),

(2) puis à saponifier ces énantiomères ester pour obtenir respectivement l'énantiomère erythro (+) [IaA(+)] et l'énantiomère érythro (-) [IaA(-)].

**11.** Procédé de préparation de l'énantiomère thréo (+) [IaB(+)] et de l'énantiomère thréo (-) [IaB(-)], correspondant à la formule (I) selon la revendication 1 et à la structure particulière (Ia) définie à la revendication 8, caractérisé en ce qu'il consiste :

(1) à séparer par chromatographie sur colonne de silice sous moyenne pression le couple racémique de diastéréoisomères thréo correspondant à la structure :

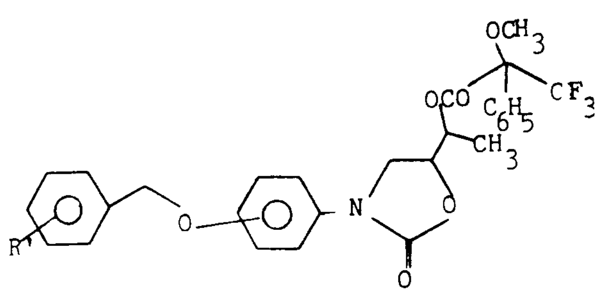

I"aB(±)

où R' à la même signification que dans la revendication 8, ce qui conduit à deux produits, le produit le moins polaire étant constitué par l'énantiomère ester I''aB(+) et le plus polaire par l'énantiomère ester I''aB(-), puis

(2) à saponifier ces énantiomères ester pour obtenir respectivement l'énantiomère thréo (+) [IaB-(+)] et l'énantiomère thréo (-) [IaB(-)].

**12.** Procédé de préparation de l'énantiomère érythro (+), de l'énantiomère érythro (-), de l'énantiomère thréo (+) et de l'énantiomère thréo (-), correspondant à la formule (I) selon la revendication 1 et à la structure particulière :

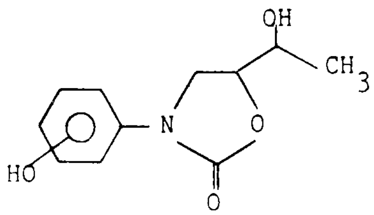

(Ib)

caractérisé en ce qu'il consiste à débenzyler respectivement l'énantiomère érythro (+), l'énantiomère érythro (-), l'énantiomère thréo (+) et l'énantiomère thréo (-), correspondant à la formule (I) selon la revendication 1 et de structure particulière (Ia) définie à la revendication 8.

**13.** Procédé de préparation de l'énantiomère érythro (+), de l'énantiomère érythro (-), de l'énantiomère thréo (+) et de l'énantiomère thréo (-), correspondant à la formule (I) selon la revendication 1 et à la structure particulière :

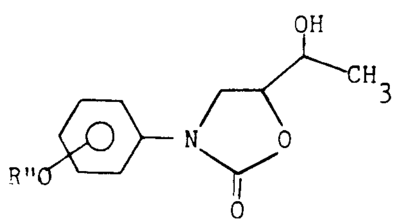

(Ic)

où R'' représente :

- un groupe alkyle linéaire ou ramifié, comportant de 1 à 6 atomes de carbone ; ou
- un groupe cycloalkylalkyle comportant de 4 à 7 atomes de carbone,

caractérisé en ce qu'il consiste à soumettre respectivement l'énantiomère érythro (+), l'énantiomère érythro (-), l'énantiomère thréo (+) et l'énantiomère thréo (-), correspondant à la formule (I) selon la revendication 1 et à la structure particulière (Ib) définie à la revendication 12, à l'action d'un agent d'alkylation de formule :

R'' - X (VIII)

où R'' a la même signification que dans la formule (Ic) et X représente un groupe bon partant, pour alkyler sélectivement le radical hydroxyle phénolique desdits énantiomères.

**14.** Mélange de 4 stéréoisomères de formule :

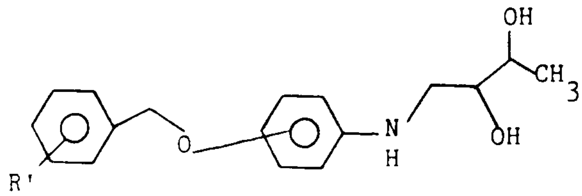

(IV)

où R' représente un atome d'hydrogène, un ou deux atomes d'halogène ou un groupement alkoxy de 1 à 4 atomes de carbone ou trifluorométhyle.

**15.** Couple racémique de diastéréoisomères érythro de formule :

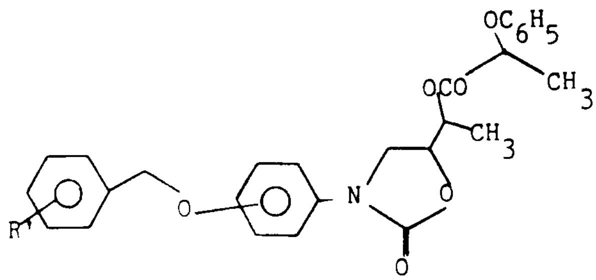

I'aA(±)

où R' représente un atome d'hydrogène, un ou deux atomes d'halogène ou un groupement alkoxy de 1 à 4 atomes de carbone ou trifluorométhyle,
ainsi que l'énantiomère ester érythro (+) et l'énantiomère ester érythro (-) constituant ce couple.

**16.** Couple racémique de diastéréoisomères thréo de formule :

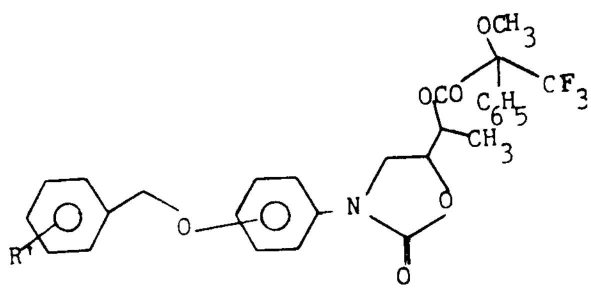

I"aB(±)

où R' représente un atome d'hydrogène, un ou deux atomes d'halogène ou un groupement alkoxy de 1 à 4 atomes de carbone ou trifluorométhyle,
ainsi que l'énantiomère ester thréo (+) et l'énantiomère ester thréo (-) constituant ce couple.

**Revendications pour les Etats contractants suivants : ES, GR**

**1.** Procédé de préparation du mélange des 4 stéréoisomères répondant à la formule :

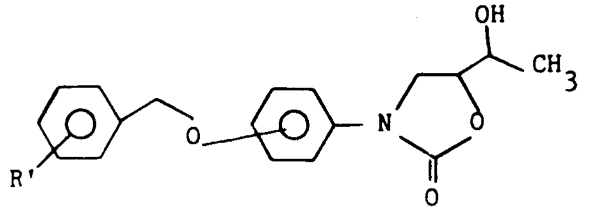

**(Ia)**

où R' représente un atome d'hydrogène, un ou deux atomes d'halogène, un groupe alkoxy de 1 à 4 atomes de carbone ou un groupe trifluorométhyle, caractérisé en ce qu'il consiste à soumettre le

mélange des 4 stéréoisomères de formule :

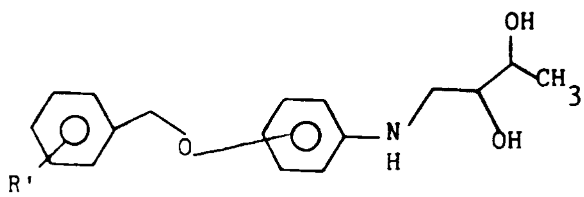

(IV)

où R' à la même signification que dans la formule (Ia), à une cyclisation par action du carbonate d'éthyle en presence d'éthylate de sodium.

2. Procédé de preparation du couple racémique de diastéréoisomères érythro et du couple racémique de diastéréoisomères thréo, correspondant à la formule (Ia) selon la revendication 1, caractérisé en ce qu'il consiste à soumettre le mélange des 4 stéréoisomères de formule (Ia) définie à la revendication 1, à une chromatographie sur colonne de silice sous moyenne pression, ce qui permet d'isoler deux produits, le produit le moins polaire correspondant au couple racémique de diastéréoisomères érythro [IaA(±)] et le produit le plus polaire correspondant au couple racémique de diastéréoisomères thréo [IaB(±)].

3. Procédé de préparation de l'énantiomère érythro (+) [IaA(+)] et de l'énantiomère érythro (-) [IaA(-)], correspondant à la formule (Ia) definie à la revendication 1, caractérisé en ce qu'il consiste :
(1) à séparer par chromatographie sur colonne de silice sous moyenne pression le couple racémique de diastéréoisomères érythro correspondant à la structure :

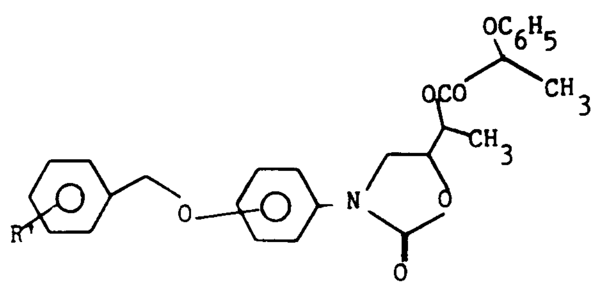

I'aA(±)

où R' a la même signification que dans la revendication 1, ce qui conduit à deux produits, le produit le moins polaire étant constitué par l'énantiomère ester I'aA(+) et le plus polaire par l'énantiomère ester I'aA(-),
(2) puis a saponifier ces énantiomères ester pour obtenir respectivement l'énantiomère érythro (+) [IaA(+)] et l'énantiomère érythro (-) [IaA(-)].

4. Procédé de préparation de l'énantiomère thréo (+) [(IaB(+) et de l'énantiomère thréo (-) [IaB(-)], correspondant à la formule (Ia) définie à la revendication 1, caractérisé en ce qu'il consiste :
(1) à séparer par chromatographie sur colonne de silice sous moyenne pression le couple racémique de diastéréoisomères thréo corrrespondant à la structure :

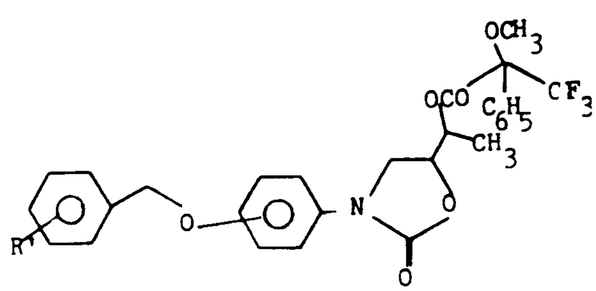

I"aB(±)

où R' a la même signification que dans la revendication 1, ce qui conduit à deux produits, le produit le moins polaire étant constitué par l'énantiomère ester I''aB(+) et le plus polaire par l'énantiomère ester I''aB(-), puis

(2) à saponifier ces énantiomères ester pour obtenir respectivement l'énantiomère thréo (+) [IaB-(+)] et l'énantiomère thréo (-) [IaB(-)].

**5.** Procédé de préparation de l'énantiomère érythro (+), de l'énantiomère érythro (-), de l'énantiomère thréo (+) et de l'énantiomère thréo (-), correspondant à la formule :

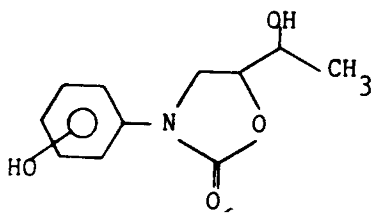

(Ib)

à l'exclusion de l'(hydroxy-4 phényl)-3 (hydroxy-1 éthyl)-5 oxazolidinone-2, caractérisé en ce qu'il consiste à débenzyler respectivement l'énantiomère érythro (+), l'énantiomère érythro (-), l'énantiomère thréo (+) et l'énantiomère thréo (-), correspondant à la formule (Ia) définie à la revendication 1.

**6.** Procédé de préparation de l'énantiomère érythro (+), de l'énantiomère érythro (-), de l'énantiomère thréo (+) et de l'énantiomère thréo (-), correspondant à la formule :

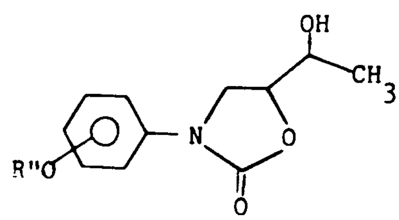

(Ic)

où R'' représente :

- un groupe alkyle linéaire ou ramifié, comportant de 1 à 6 atomes de carbone ; ou
- un groupe cycloalkylalkyle comportant de 4 à 7 atomes de carbone,

caractérisé en ce qu'il consiste à soumettre respectivement l'énantiomère erythro (+), l'énantiomère érythro (-), l'énantiomère thréo (+) et l'énantiomère thréo (-), correspondant à la formule (Ib) définie à la revendication 5, à l'action d'un agent d'alkylation de formule :

R'' - X (VIII)

ou R'' a la même signification que dans la formule (Ic) et X représente un groupe bon partant, pour alkyler sélectivement le radical hydroxyle phénolique desdits énantiomères.

**7.** Procédé selon l'une des revendications 1 à 4, caractérisé en ce que le reste :

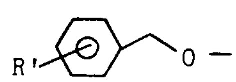

est en position para et R' = H ; 3-F ; 3-Cl ; 4-F ; 3-F, 4-Cl ; 3-$OCH_3$ ; 3-$CF_3$.

**8.** Procédé selon la revendication 6, caractérisé en ce que R'' = méthyle, butyle, i-pentyle, éthyl-2 butyle, cyclohexylméthyle.

**9.** Procédé de préparation d'une composition à usage thérapeutique, caractérisé en ce qu'il consiste à mélanger (a) un composé pouvant être obtenu par un procédé selon l'une des revendications

précédentes, et (b) un véhicule physiologiquement acceptable pour ce composé.

**10.** Utilisation du mélange des 4 stéréoisomers de formule (I) ci-dessous, de chacun des couples racémiques de diastéréoisomères correspondants et de chacun des énantiomères correspondant à chaque couple :

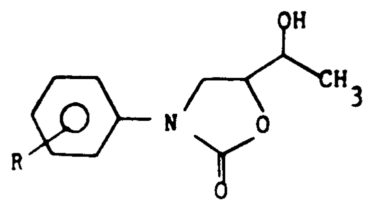

(I)

dans laquelle R représente :
- le groupe benzyloxy ;
- le groupe benzyloxy substitué par un ou deux atomes d'halogène ; par un groupement alkoxy comportant de 1 à 4 atomes de carbone ; ou par un groupement trifluorométhyle ;
- un radical hydroxy ;
- un groupe alkoxy linéaire ou ramifié, comprenant de 1 à 6 atomes de carbone ; ou
- un groupe cycloalkylalkyloxy comprenant de 4 à 7 atomes de carbone à l'exclusion de l'-(hydroxy-4 phényl)-3 (hydroxy-1 éthyl)-5 oxazolidinone-2, pour la préparation d'un agent inhibiteur de la monoamine oxydase, en particulier de type B.

**Claims**
**Claims for the following Contracting States : BE, CH, DE, GB, IT, LI, LU, NL, SE**

**1.** A mixture of the 4 stereoisomers of formula (I) below, each corresponding racemic pair of diastereoisomers and each of the enantiomers in each pair:

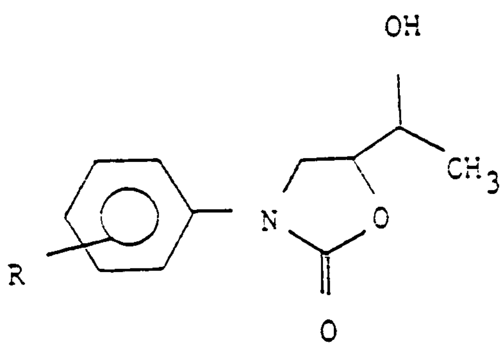

(I)

in which R represents:
- a benzyloxy group;
- a benzyloxy group substituted by one or two halogen atoms; by an alkoxy group containing 1 to 4 carbon atoms; or by a trifluoromethyl group;
- a hydroxy radical;
- a linear or branched alkoxy group having 1 to 6 carbon atoms;
  or
- a cycloalkylalkyloxy group having 4 to 7 carbon atoms, except the 3-(4-hydroxy phenyl)-5-(1-hydroxy ethyl)-2-oxazolidinone.

**2.** The mixture, pairs and enantiomers as in Claim 1, in which R is in the para position and represents a benzyloxy group or a benzyloxy group substituted with 3-F; 3-Cl; 4-F; 3-F, 4-Cl; 3-OCH$_3$; or 3-CF$_3$.

3. The mixture, pairs and enantiomers as in Claim 1, in which R is in the para position and represents the methoxy, butoxy, iso-pentoxy, ethyl-2-butoxy or cyclohexylmethyloxy group.

4. Enantiomers as in any of Claims 1 to 3, having the threo relative configuration.

5. Enantiomers as in Claim 4, having the absolute configuration (R, R).

6. A medicament having a notable inhibitive action on monoamine oxydase, more particularly type B thereof, characterized in that it consists of a mixture, pair or enantiomer as specified in any of Claims 1 to 5.

7. A pharmaceutical preparation having an inhibitive action on monoamine oxydase, more particularly type B thereof, characterized in that it consists of a mixture, pair or enantiomer as specified in any of Claims 1 to 5, in association with an acceptable pharmaceutical vehicle.

8. A process for the preparation of the mixture of the 4 stereoisomers of formula (I) as in Claim 1, with the specific structure:

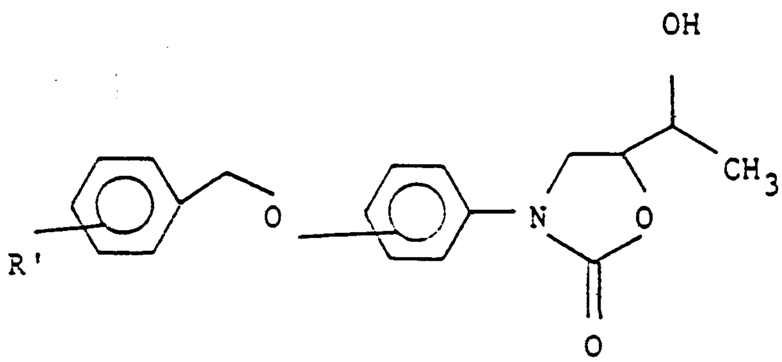

(Ia)

in which R' represents a hydrogen atom, one or two halogen atoms, an alkoxy group having 1 to 4 carbon atoms or a trifluoromethyl group; characterized in that the mixture of 4 stereoisomers of the formula

(IV)

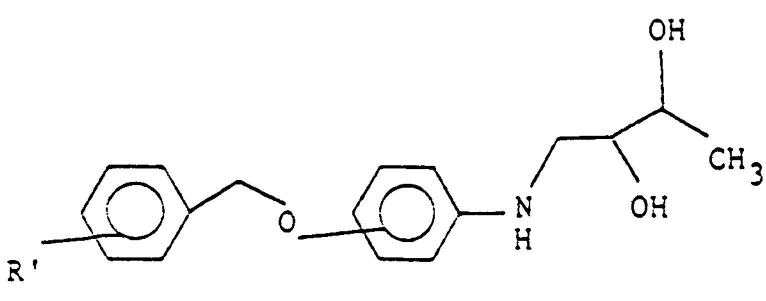

in which R' has the same signification as in formula (Ia), is subjected to cyclisation by treatment with ethyl carbonate in the presence of sodium ethylate.

9. A process for the preparation of the racemic pair of erythro diastereoisomers and the racemic pair of threo diastereoisomers, corresponding to formula (I) as in Claim 1 and having the specific structure (Ia) as defined in Claim 8, characterized in that the mixture of 4 stereoisomers having the formula (I) and the specific structure (Ia) defined in Claim 8 is subjected to chromatography on a silica column under medium pressure, so as to separate two products, the less polar product corresponding to the racemic pair of erythro diastereoisomers [IaA (±)] and the more polar product to the racemic pair of threo diastereoisomers [IaB (±)].

**10.** A process for the preparation of the erythro (+) enantiomer [IaA (+)] and the erythro (-) enantiomer [IaA (-)] corresponding to formula (I) in Claim 1 and having the particular structure (Ia) defined in Claim 8, characterized in that:

(1) the racemic pair of erythro diastereoisomers corresponding to the structure:

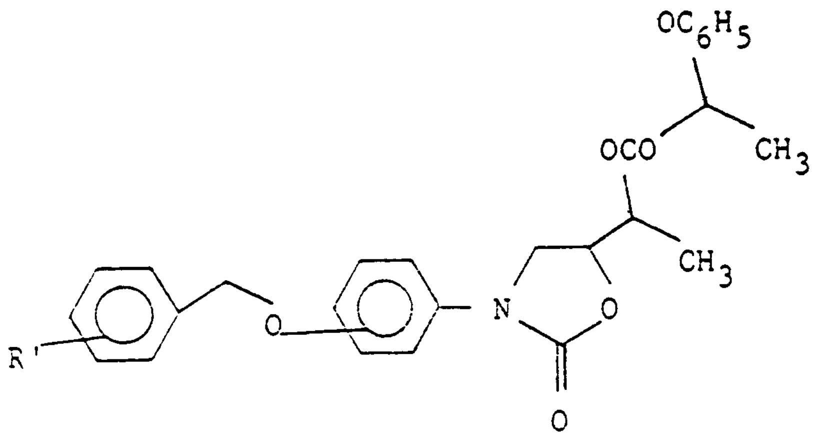

I'aA(±)

in which R' has the same signification as in Claim 8, is separated chromatographically on a silica column under medium pressure into two products, the less polar of which consists of the ester enantiomer I'aA(+) while the more polar consists of the ester enantiomer I'aA(-);

(2) the ester enantiomers are then saponified to isolate the erythro (+) enantiomer [IaA (+)] from the erythro (-) enantiomer [IaA (-)].

**11.** A process for the preparation of the threo (+) enantiomer [IaB (+)] and the threo (-) enantiomer [IaB (-)], corresponding to the formula (I) in claim 1 and having the particular structure (Ia) defined in Claim 8, characterized in that:

(1) the racemic pair of threo diastereoisomers corresponding to the structure:

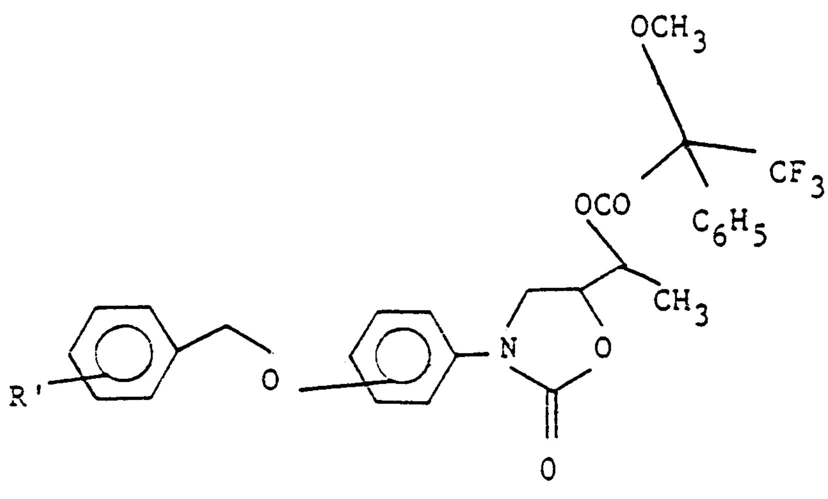

I'' aB (±)

in which R' has the same signification as in Claim 8, is separated chromatographically on a silica column under medium pressure into two products, the less polar of which consists of the ester enantiomer I'' aB(+) while the more polar consists of the enantiomer I'' aB(-);

(2) the ester enantiomers are then saponified to isolate the threo (+) enantiomer [IaB (+)] from the threo (-) enantiomer [IaB(-)].

**12.** A process for the preparation of the erythro (+) enantiomer, the erythro (-) enantiomer, the threo (+) enantiomer and the threo (-) enantiomer, corresponding to formula (I) in Claim 1 and having the

particular structure:

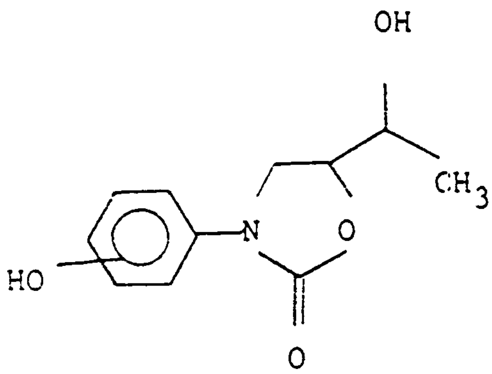

(Ib)

characterized in that the erythro (+) enantiomer, the erythro (-) enantiomer, the threo (+) enantiomer and the threo (-) enantiomer, corresponding to formula (I) in Claim 1 and having the particular structure defined in claim 8 are debenzylated.

**13.** A process for the preparation of the erythro (+) enantiomer, the erythro (-) enantiomer, the threo (+) enantiomer and the threo (-) enantiomer, corresponding to formula (I) in Claim 1 and having the particular structure:

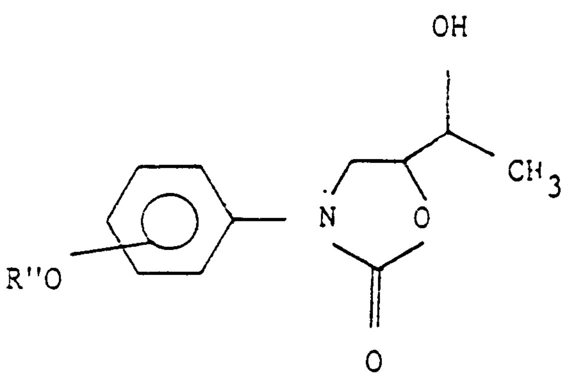

(Ic)

in which R" represents:

- a linear or branched alkyl group having 1 to 6 carbon atoms; or
- a cycloalkylalkyl group having 4 to 7 carbon atoms,

characterized in that the erythro (+) enantiomer, the erythro (-) enantiomer, the threo (+) enantiomer and the threo (-) enantiomer, corresponding to formula (I) of Claim 1 and having the particular structure (Ib) defined in Claim 12 are respectively treated with an alkylating agent having the formula:

R" - X (V III)

in which R" has the same signification as in formula (Ic), while X represents a good leaving group, thereby selectively alkylating the phenolic hydroxyl radical in the said enantiomers.

**14.** A mixture of the 4 stereoisomers having the formula:

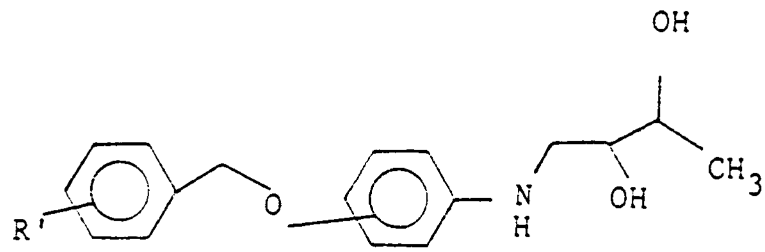

(IV)

in which R' represents a hydrogen atom, one or two halogen atoms, an alkoxy group having 1 to 4 carbon atoms or a trifluoromethyl group.

**15.** A racemic pair of erythro diastereoisomers having the formula:

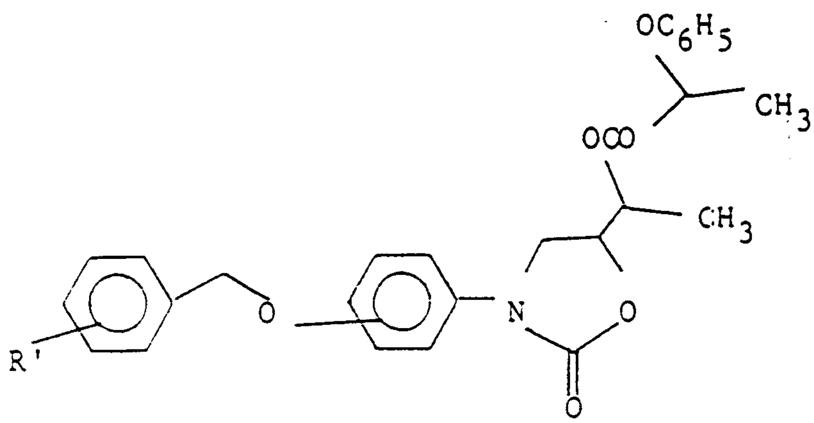

I'aA(±)

in which R' represents a hydrogen atom, one or two halogen atoms, an alkoxy group having 1 to 4 carbon atoms or a trifluoromethyl group, as well as the erythro (+) ester enantiomer and the erythro (-) ester enantiomer making up the said pair.

**16.** A racemic pair of threo diastereoisomers having the formula:

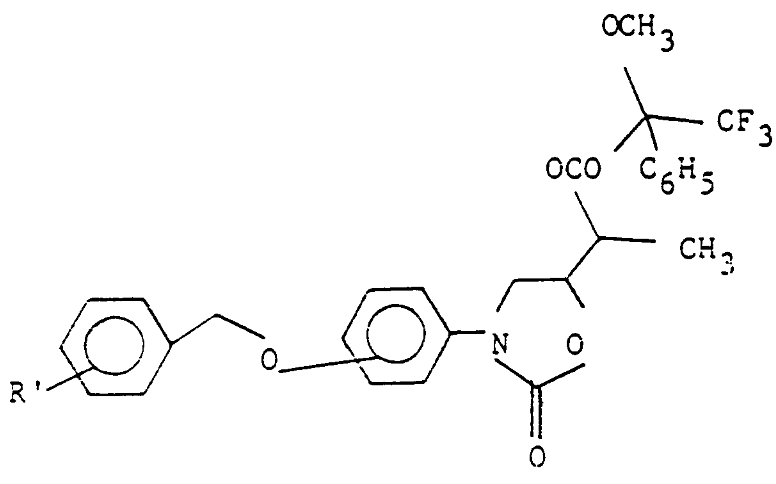

I"aB(±)

in which R' represents a hydrogen atom, one or two halogen atoms, an alkoxy group having 1 to 4 carbon atoms or a trifluoromethyl group, as well as the threo (+) ester enantiomer and the threo (-) ester enantiomer making up the said pair.

**Claims for the following Contracting States : ES, GR**

**1.** A process for the preparation of the mixture of the 4 stereoisomers of formula:

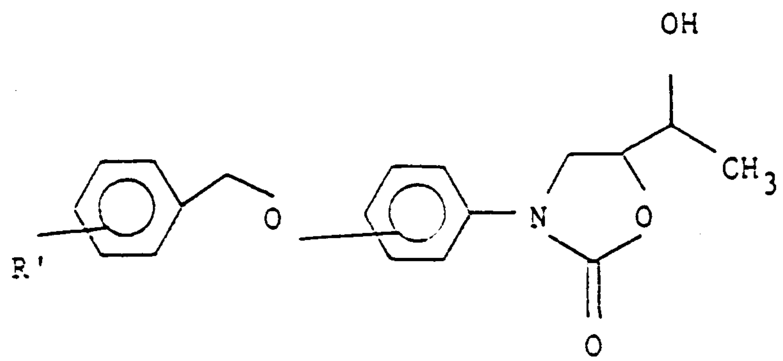

(Ia)

in which R' represents a hydrogen atom, one or two halogen atoms, an alkoxy group having 1 to 4 carbon atoms or a trifluoromethyl group, characterized in that the mixture of 4 stereoisomers of the formula

(IV)

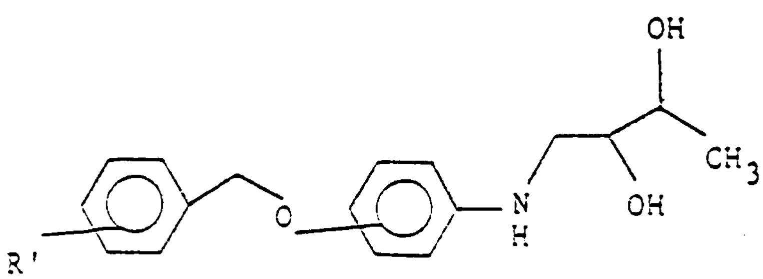

in which R' has the same signification as in formula (Ia), is subjected to cyclisation by treatment with ethyl carbonate in the presence of sodium ethylate.

**2.** A process for the preparation of the racemic pair of erythro diastereoisomers and the racemic pair of threo diastereoisomers, corresponding to formula (Ia) as in Claim 1, characterized in that the mixture of the 4 stereoisomers having the formula (Ia) defined in Claim 1 is subjected to chromatography on a silica column under medium pressure, so as to separate two product, the less polar product corresponding to the racemic pair of erythro diastereoisomers [IaA (±)] and the more polar products to the racemic pair of threo diastereoisomers [IaB (±)].

**3.** A process for the preparation of the erythro (+) enantiomer [IaA (+)] and the erythro (-) enantiomer [IaA (-)] corresponding to formula (Ia) defined in Claim 1, characterized in that:
(1) the racemic pair of erythro diastereoisomers corresponding to the structure:

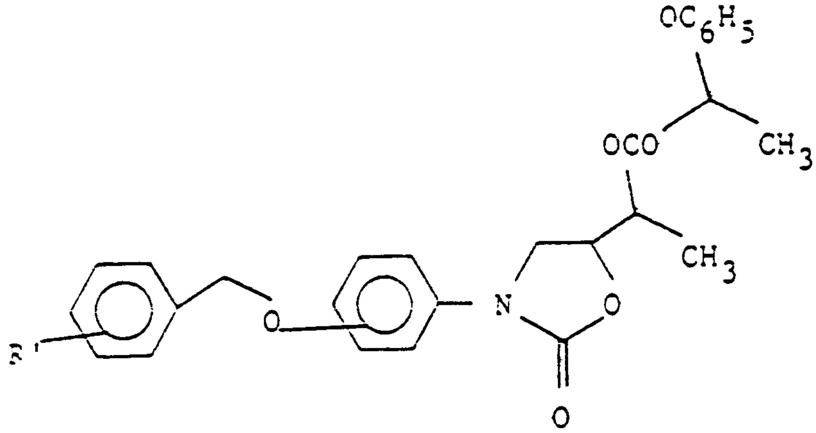

I'aA(±)

in which R' has the same signification as in Claim 1, is separated chromatographically on a silica column under medium pressure into two products, the less polar of which consists of the ester enantiomer I'aA(+) while the more polar consists of the ester enantiomer I'aA(-) ;
(2) the ester enantiomers are then saponified to isolate the erythro (+) enantiomer [IaA (+)] from the erythro (-) enantiomer [IaA (-)].

**4.** A process for the preparation of the threo (+) enantiomer [IaB (+)] and the threo (-) enantiomer [IaB (-)], corresponding to the formula (Ia) defined in Claim 1, characterized in that:
(1) the racemic pair of threo diastereoisomers corresponding to the structure:

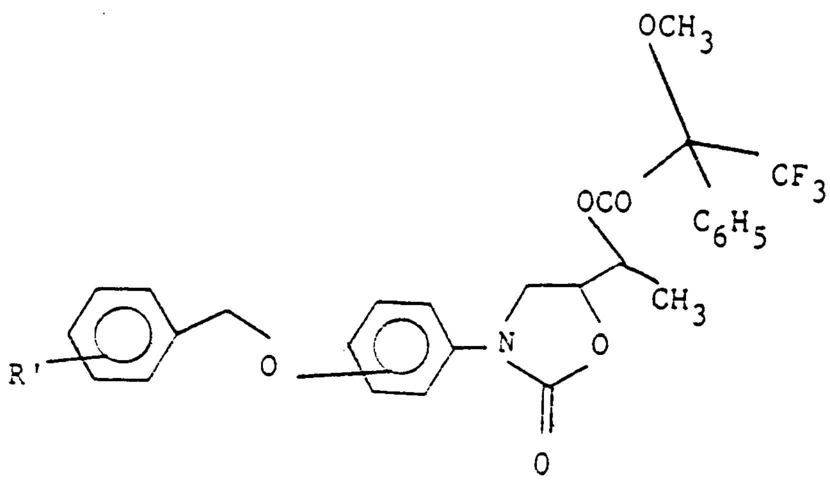

(I'' aB (±)

in which R' has the same signification as in Claim 1, is separated chromatographically on a silica column under medium pressure into two products, the less polar of which consists of the ester enantiomer I'' aB(+) while the more polar consists of the ester enantiomer I''aB(-);
(2) the ester enantiomers are then saponified to isolate the threo (+) enantiomer [IaB (+)] from the threo (-) enantiomer [IaB (-)].

**5.** A process for the preparation of the erythro (+) enantiomer, the erythro (-) enantiomer, the threo (+) enantiomer and the threo (-) enantiomer, corresponding to formula:

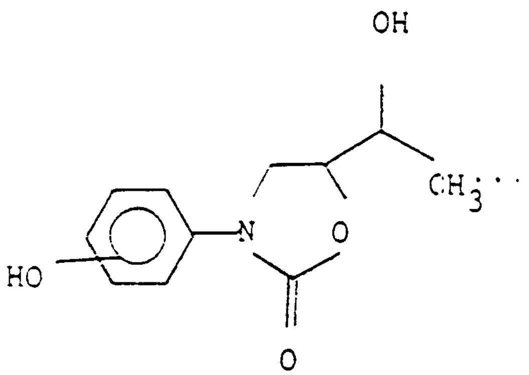

(Ib)

with the exclusion of the 3-(4-hydroxyphenyl)-5-(1-hydroxyethyl)-2-oxazolidinone, characterized in that the erythro (+) enantiomer, the erythro (-) enantiomer, the threo (+) enantiomer and the threo (-) enantiomer, corresponding to formula (Ia) defined in Claim 1 are debenzylated.

**6.** A process for the preparation of the erythro (+) enantiomer, the erythro (-) enantiomer, the threo (+) enantiomer and the threo (-) enantiomer, corresponding to formula:

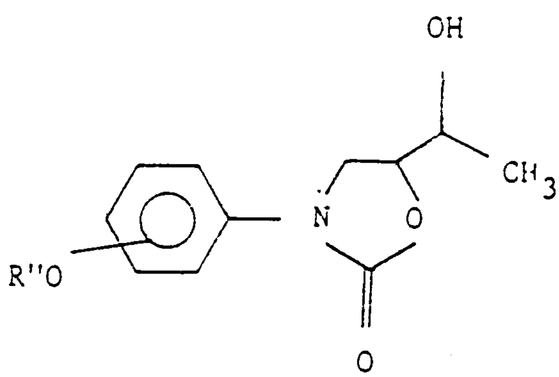

(Ic)

in which R'' represents:

- a linear or branched alkyl group having 1 to 6 carbon atoms; or
- a cycloalkylalkyl group having 4 to 7 carbon atoms,

characterized in that the erythro (+) enantiomer, the erythro (-) enantiomer, the threo (+) enantiomer and the threo (-) enantiomer, corresponding to formula (Ib) defined in Claim 5 are respectively treated with an alkylating agent having the formula:

R'' - X (VIII)

in which R'' has the same signification as in formula (Ic), while X represents a good leaving group, thereby selectively alkylating the phenolic hydroxyl radical in the said enantiomers.

**7.** The process according to one of claims 1 to 4, characterized in that the residue of formula:

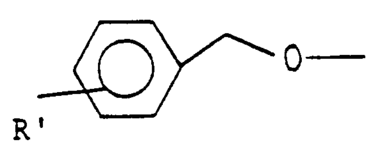

is in para position and R' = H; 3-F; 3-Cl; 4-F; 3-F, 4-Cl; 3-$OCH_3$; 3-$CF_3$.

**8.** The process according to claim 6, characterized in that R'' = methyl, butyl, i-pentyl, 2-ethylbutyl, cyclohexymethyl.

9. A process for preparing a therapeutical composition, characterized in that it consists in mixing (a) a compound which may be obtained by a process according to one of the preceding Claims, and (b) a physiologically acceptable vehicle for this compound.

10. Use of the mixture of the 4 stereoisomers of formula (I) below, of each of the corresponding racemic pairs of diastereoisomers and of each of the enantiomers in each pair:

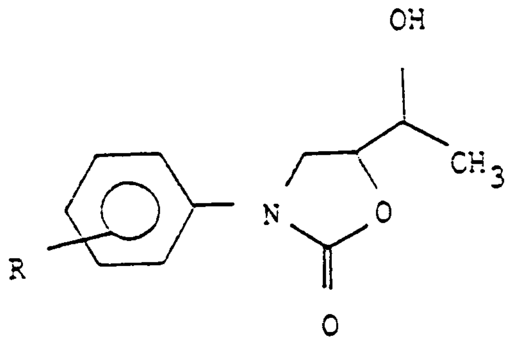

(I)

in which R represents:
- a benzyloxy group;
- a benzyloxy group substituted by one or two halogen atoms; by an alkoxy group containing 1 to 4 carbon atoms; or by a trifluoromethyl group;
- a hydroxy radical;
- a linear or branched alkoxy group having 1 to 6 carbon atoms; or
- a cycloalkylalkyloxy group having 4 to 7 carbon atoms, with the exclusion of the 3-(4-hydroxyphenyl)-5-(1-hydroxyethyl)-2-oxazolidinone, for the preparation of an inhibitor of the monoamine oxidase, especially of the B-type.

**Patentansprüche**
**Patentansprüche für folgende Vertragsstaaten : BE, CH, DE, GB, IT, LI, LU, NL, SE**

1. Mischung der 4 Stereoisomeren der nachstehenden Formel (I), jedes der entsprechenden racemischen Diastereomerenpaare und jedes der jedem Paar entsprechenden Enantiomeren:

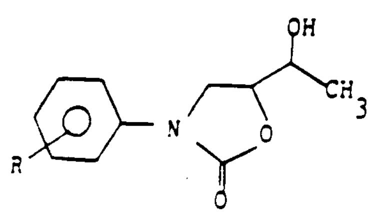

(I)

worin R bedeutet:
die Benzyloxygruppe;
die Benzyloxygruppe, substituiert durch ein oder zwei Halogenatome; durch eine Alkoxygruppe mit 1 bis 4 Kohlenstoffatomen; oder durch eine Trifluormethylgruppe;
ein Hydroxyradikal;
eine geradkettige oder verzweigte Alkoxygruppe mit 1 bis 6 Kohlenstoffatomen; oder
eine Cycloalkylalkyloxygruppe mit 4 bis 7 Kohlenstoffatomen, unter Ausschluß des 3-(4-Hydroxyphenyl)-5-(1-hydroxy-ethyl)-2-oxazolidinons.

2. Mischung, Paare und Enantiomere nach Anspruch 1, in denen R in para-Position ist und die Benzyloxygruppe oder eine Benzyloxygruppe, substituiert durch 3-F; 3-Cl; 4-F; 3-F, 4-Cl; 3-$OCH_3$; oder 3-$CF_3$ bedeutet.

3. Mischung, Paare und Enantiomere nach Anspruch 1, in denen R in para-Position ist und die Methoxy-, Butoxy-, i-Pentoxy-, 2-Ethylbutoxy- oder Cyclohexylmethyloxygruppe bedeutet.

4. Enantiomere nach einem der Ansprüche 1 bis 3, mit der relativen Konfiguration threo.

5. Enantiomere nach Anspruch 4, mit der absoluten Konfiguration (R, R).

6. Arzneimittel, welches insbesondere eine hemmende Wirkung auf die Monoaminoxidase, insbesondere des Typs B, besitzt, **dadurch gekennzeichnet,** daß es aus der Mischung, einem Paar oder einem Enantiomer nach einem der Ansprüche 1 bis 5 zusammengesetzt ist.

7. Pharmazeutische Zusammensetzung, welche eine hemmende Wirkung auf die Monoaminoxidase, insbesondere des Typs B, besitzt, **dadurch gekennzeichnet,** daß sie die Mischung, ein Paar oder ein Enantiomer nach einem der Ansprüche 1 bis 5 in Verbindung mit einem pharmazeutisch annehmbaren Träger umfaßt.

8. Verfahren zur Herstellung der Mischung der vier Stereoisomeren der Formel (I) nach Anspruch 1 und der speziellen Struktur:

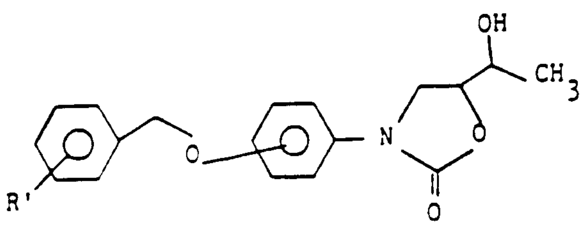

(Ia)

worin R' ein Wasserstoffatom, ein oder zwei Halogenatome oder eine Alkoxygruppe mit 1 bis 4 Kohlenstoffatomen oder Trifluormethyl bedeutet, **dadurch gekennzeichnet,** daß es das Unterwerfen der Mischung der vier Stereoisomeren der Formel:

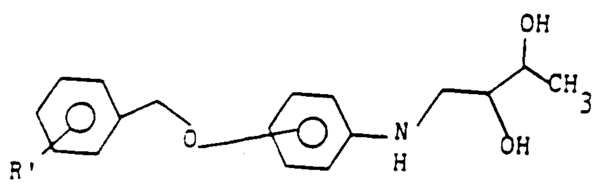

(IV)

worin R' die gleiche Bedeutung wie in der Formel (Ia) hat, einer Cyclisierung mittels Ethylcarbonat in Gegenwart von Natriumethylat beinhaltet.

9. Verfahren zur Herstellung des racemischen erythro-Diastereomeren-Paares und des racemischen threo-Diastereomeren-Paares, entsprechend der Formel (I) nach Anspruch 1 und der speziellen Struktur (Ia), die im Anspruch 8 definiert ist, **dadurch gekennzeichnet,** daß es das Unterwerfen der Mischung der vier Stereoisomeren der Formel (I) und der speziellen Struktur (Ia), die in Anspruch 8 definiert ist, einer Chromatographie über eine Kieselerdesäule unter mittlerem Druck beinhaltet, was die Isolierung von zwei Produkten ermöglicht, wobei das weniger polare Produkt dem racemischen erythro-Diastereomeren-Paar [IaA(±)] und das stärker polare Produkt dem racemischen threo-Diastereomeren-Paar [IaB(±)] entspricht.

10. Verfahren zur Herstellung des erythro-(+)-Enantiomers [IaA(+)] und des erythro-(-)-Enantiomers [IaA(-)), entsprechend der Formel (I) nach Anspruch 1 und der speziellen Struktur (Ia), die in Anspruch 8 definiert ist, **dadurch gekennzeichnet,** daß es beinhaltet:

(1) Das Trennen des racemischen erythro-Diastereomeren-Paares entsprechend der Struktur:

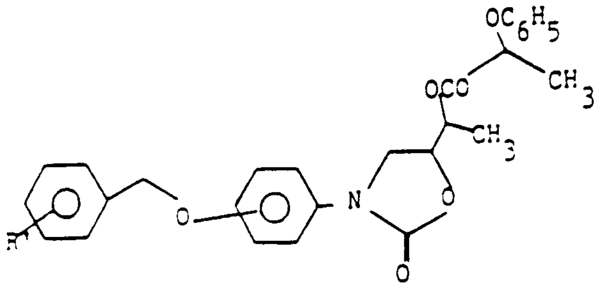

I'aA(±)

worin R' die gleiche Bedeutung wie in Anspruch 8 hat, durch Chromatographie über eine Kieselerdesäule unter mittlerem Druck, was zu zwei Produkten führt, wobei das weniger polare Produkt aus dem Enantiomeren-Ester I'aA(+) und das stärker polare Produkt aus dem Enantiomeren-Ester I'aA(-) besteht,

(2) das anschließende Verseifen dieser Enantiomeren-Ester, um das erythro-(+)-Enantiomer [IaA(+)] bzw. das erythro-(-)-Enantiomer [IaA(-)] zu erhalten.

**11.** Verfahren zur Herstellung des threo-(+)-Enantiomers [IaB(+)] und des threo-(-)-Enantiomers [IaB(-)], entsprechend der Formel (I) nach Anspruch 1 und der speziellen Struktur (Ia), die im Anspruch 8 definiert ist, **dadurch gekennzeichnet,** daß es beinhaltet:

(1) das Trennen des racemischen threo-Diastereomeren-Paares entsprechend der Formel

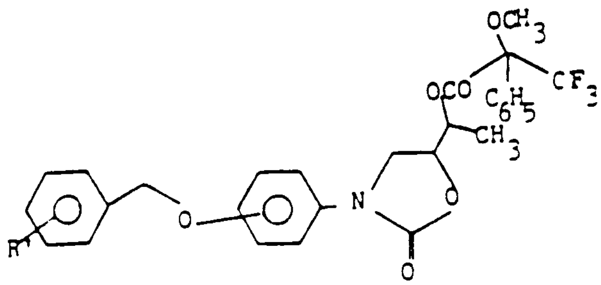

I"aB(±)

worin R' die gleiche Bedeutung wie in Anspruch 8 hat, durch Chromatographie über eine Kieselerdesäule unter mittlerem Druck, was zu zwei Produkten führt, wobei das weniger polare Produkt aus dem Enantiomeren-Ester I"aB(+) und das stärker polare Produkt aus dem Enantiomeren-Ester I"aB(-) besteht, und anschließend

(2) das Verseifen dieser Enantiomeren-Ester, um jeweils das threo-(+)-Enantiomer [IaB(+)] bzw. das threo-(-)-Enantiomer [IaB(-)] zu erhalten.

**12.** Verfahren zur Herstellung des erythro-(+)-Enantiomers des erythro-(-)-Enantiomers, des threo-(+)-Enantiomers und des threo-(-)-Enantiomers, entsprechend der Formel (I) nach Anspruch 1 und der speziellen Struktur:

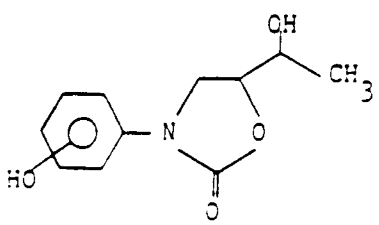

(Ib)

**dadurch gekennzeichnet,** daß es darin besteht, jeweils das erythro-(+)-Enantiomer, das erythro-(-)-Enantiomer, das threo-(+)-Enantiomer bzw. das threo-(-)-Enantiomer, entsprechend der Formel (I) nach Anspruch 1 und der speziellen Struktur (Ia), die im Anspruch 8 definiert ist, zu debenzylieren.

**13.** Verfahren zur Herstellung des erythro-(+)-Enantiomers, des erythro-(-)-Enantiomers, des threo-(+)-Enantiomers und des threo-(-)-Enantiomers, entsprechend der Formel (I) nach Anspruch 1 und der speziellen Struktur:

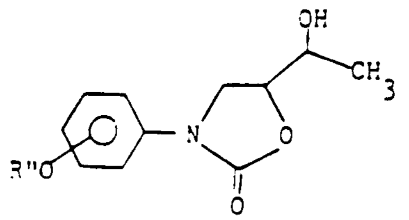

(Ic)

worin R" bedeutet:
eine geradkettige oder verzweigte Alkylgruppe mit 1 bis 6 Kohlenstoffatomen; oder
eine Cycloalkylalkylgruppe mit 4 bis 7 Kohlenstoffatomen, **dadurch gekennzeichnet,** daß es das Unterwerfen des erythro-(+)-Enantiomers, des erythro-(-)-Enantiomers, des threo-(+)-Enantiomers bzw. des threo-(-)Enantiomers, entsprechend der Formel (I) nach Anspruch 1 und der speziellen Struktur (Ib), die im Anspruch 12 definiert ist, der Wirkung eines Alkylierungsmittels mit der Formel:

R" - X (VIII)

worin R" die gleiche Bedeutung wie in der Formel (Ic) hat und X eine gute Abgangsgruppe bedeutet, um selektiv das phenolische Hydroxylradikal der genannten Enantiomeren zu alkylieren, beinhaltet.

**14.** Mischung der vier Stereoisomeren der Formel:

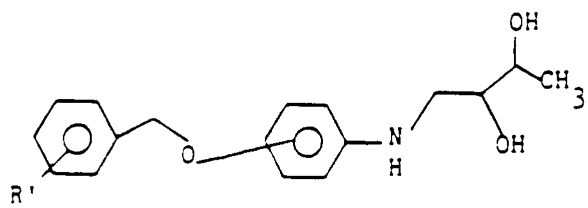

(IV)

worin R' ein Wasserstoffatom, ein oder zwei Halogenatome oder eine Alkoxygruppe mit 1 bis 4 Kohlenstoffatomen oder Trifluormethyl bedeutet.

**15.** Racemisches erythro-Diastereomeren-Paar der Formel:

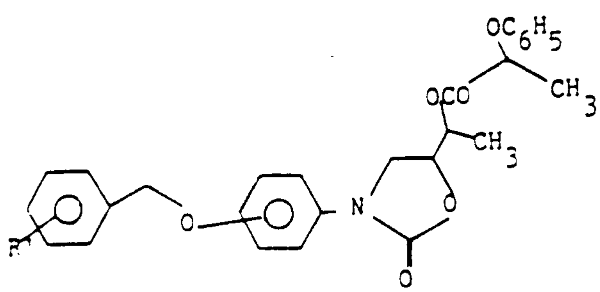

I'aA(±)

worin R' ein Wasserstoffatom, ein oder zwei Halogenatome oder eine Alkoxygruppe mit 1 bis 4 Kohlenstoffatomen oder Trifluormethyl bedeutet, ebenso wie den erythro-(+)-Enantiomeren-Ester und den erythro-(-)-Enantiomeren-Ester, aus denen dieses Paar besteht.

**16.** Racemisches threo-Diastereomeren-Paar der Formel

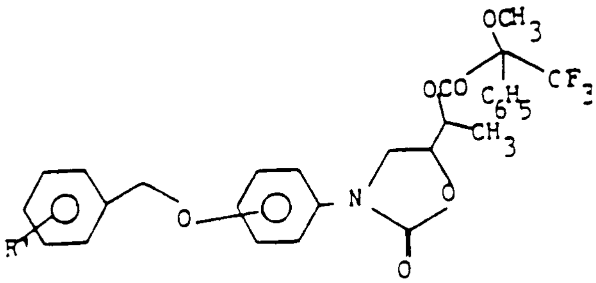

I"aB(±)

worin R' ein Wasserstoffatom, ein oder zwei Halogenatome oder eine Alkoxygruppe mit 1 bis 4 Kohlenstoffatomen oder Trifluormethyl bedeutet, ebenso wie den threo-(+)-Enantiomeren-Ester und den threo-(-)-Enantiomeren-Ester, aus denen dieses Paar besteht.

**Patentansprüche für folgende Vertragsstaaten : ES, GR**

**1.** Verfahren zur Herstellung der Mischung der vier Stereoisomeren der Formel:

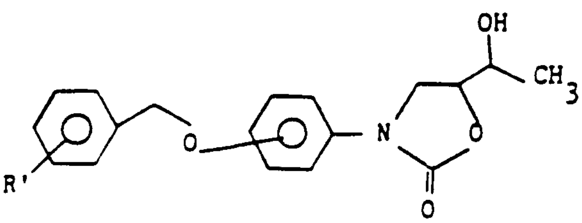

(Ia)

worin R' ein Wasserstoffatom, ein oder zwei Halogenatome, eine Alkoxygruppe mit 1 bis 4 Kohlenstoffatomen oder eine Trifluormethylgruppe bedeutet, **dadurch gekennzeichnet,** daß es das Unterwerfen der Mischung der vier Stereoisomeren der Formel:

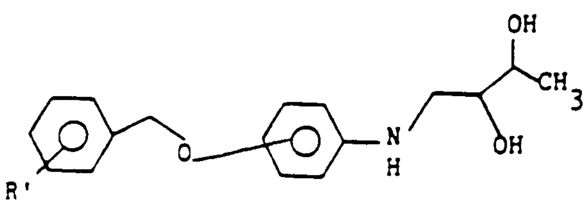

(IV)

worin R' die gleiche Bedeutung wie in der Formel (Ia) hat, einer Cyclisierung durch Ethylcarbonat in Gegenwart von Natriumethylat beinhaltet.

**2.** Verfahren zur Herstellung des racemischen erythro-Diastereomeren-Paares und des racemischen threo-Diastereomeren-Paares, entsprechend der Formel (Ia) nach Anspruch 1, **dadurch gekennzeichnet,** daß es das Unterwerfen der Mischung der vier Stereoisomeren der Formel (Ia), die im Anspruch 1 definiert ist, einer Chromatographie über eine Kieselerdesäule unter mittlerem Druck beinhaltet, was die Isolierung von zwei Produkten ermöglicht, wobei das weniger polare Produkt dem racemischen erythro-Diastereomeren-Paar [IaA(±)] und das stärker polare Produkt dem racemischen threo-Diastereomeren-Paar [IaB(±)] entspricht.

**3.** Verfahren zur Herstellung des erythro-(+)-Enantiomers [IaA(+)] und des erythro-(-)-Enantiomers [IaA(-)], entsprechend der Formel (Ia), die im Anspruch 1 definiert ist**, dadurch gekennzeichnet,** daß es beinhaltet:

(1) das Trennen des racemischen erythro-Diastereomeren-Paares, welches der Struktur:

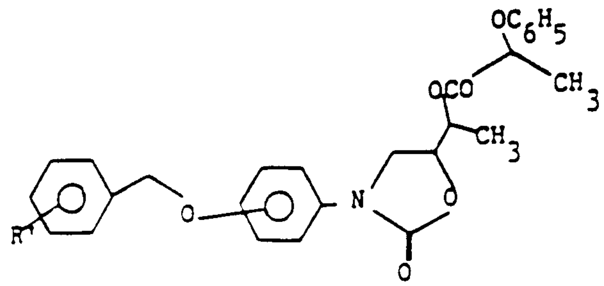

I'aA(±)

worin R' die gleiche Bedeutung wie in Anspruch 1 hat, entspricht, durch Chromatographie über eine Kieselerdesäule unter mittleren Druck, was zu zwei Produkten führt, wobei das weniger polare Produkt aus dem Enantiomeren-Ester I'aA(+) und das stärker polare Produkt aus dem Enantiomeren-Ester I'aA(-) besteht,

(2) das anschließende Verseifen dieser Enantiomeren-Ester, um das erythro-(+)-Enantiomer [IaA(+)] bzw. das erythro-(-)-Enantiomer [IaA(-)] zu erhalten.

**4.** Verfahren zur Herstellung des threo-(+)-Enantiomers [IaB(+)] und des threo-(-)-Enantiomers [IaB(-)], entsprechend der Formel (Ia), die im Anspruch 1 definiert ist, **dadurch gekennzeichnet,** daß es beinhaltet:

(1) das Trennen des racemischen threo-Diastereomeren-Paares, welches der Struktur:

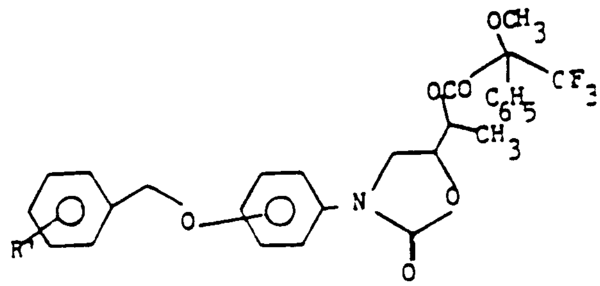

I"aB(±)

worin R' die gleiche Bedeutung wie in Anspruch 1 hat, entspricht, durch Chromatographie über eine Kieselerdesäule unter mittleren Druck, was zu zwei Produkten führt, wobei das weniger polare Produkt aus dem Enantiomeren-Ester I"aB(+) und das stärker polare aus dem Enantiomeren-Ester I"aB(-) besteht,

(2) das Verseifen dieser Enantiomeren-Ester, um jeweils das threo-(+)-Enantiomer [IaB(+)] bzw. das threo-(-)-Enantiomer [IaB(-)] zu erhalten.

**5.** Verfahren zur Herstellung des erythro-(+)-Enantiomers, des erythro-(-)-Enantiomers, des threo-(+)-Enantiomers und des threo-(-)-Enantiomers, entsprechend der Formel:

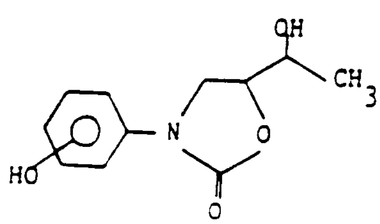

(Ib)

unter Ausschluß des 3-(4-Hydroxyphenyl)-5-(1-hydroxyethyl)-2-oxazolidinon, **dadurch gekennzeichnet,** daß es das Debenzylieren des erythro-(+)-Enantiomers, des erythro-(-)-Enantiomers, des threo-(+)-Enantiomers bzw. des threo-(-)-Enantiomers, entsprechend der Formel (Ia), die im Anspruch 1 definiert ist, beinhaltet.

6. Verfahren zur Herstellung des erythro-(+)-Enantiomers, des erythro-(-)-Enantiomers, des threo-(+)-Enantiomers und des threo-(-)-Enantiomers, entsprechend der Formel:

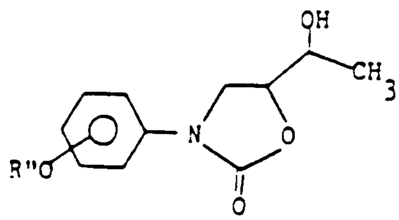

(Ic)

worin R" bedeutet:
eine geradkettige oder verzweigte Alkylgruppe mit 1 bis 6 Kohlenstoffatomen; oder
eine Cycloalkylalkylgruppe mit 4 bis 7 Kohlenstoffatomen,

**dadurch gekennzeichnet,** daß es das Unterwerfen des erythro-(+)-Enantiomers, des erythro-(-)-Enantiomers, des threo-(+)-Enantiomers bzw. des threo-(-)-Enantiomers, entsprechend der Formel (Ib), die im Anspruch 5 definiert ist, der Wirkung eines Alkylierungsmittels der Formel:

R" - X (VIII)

worin R" die gleiche Bedeutung wie in der Formel (Ic) hat und X eine gute Abgangsgruppe bedeutet, um selektiv das phenolische Hydroxylradikal der genannten Enantiomeren zu alkylieren, beinhaltet.

7. Verfahren nach einem der Ansprüche 1 bis 4**, dadurch gekennzeichnet,** daß der Rest

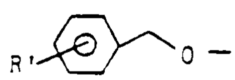

in der para-Position ist und R' = H; 3-F; 3-Cl; 4-F; 3-F, 4-Cl; 3-$OCH_3$; 3-$CF_3$.

8. Verfahren nach Anspruch 6, **dadurch gekennzeichnet,** daß R" = Methyl, Butyl, i-Pentyl, 2-Ethylbutyl, Cyclohexylmethyl.

9. Verfahren zur Herstellung einer Zusammensetzung zur therapeutischen Verwendung,
**dadurch gekennzeichnet,** daß
es das Mischen (a) einer Verbindung, die nach einem Verfahren nach einem der vorhergehenden Ansprüche erhalten werden kann und (b) eines physiologisch annehmbaren Trägers für diese Verbindung beinhaltet.

10. Verwendung der Mischung der vier Stereoisomeren der nachstehenden Formel (I), von jedem der entsprechenden racemischen Diastereomeren-Paare und von jedem der jedem Paar entsprechenden Enantiomeren:

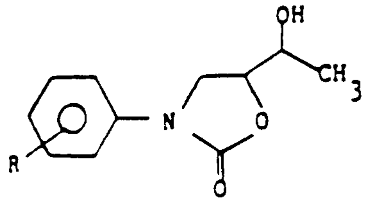

(I)

in welcher R bedeutet:
die Benzyloxygruppe;

die Benzyloxygruppe substituiert durch ein oder zwei Halogenatome; durch eine Alkoxygruppe mit 1 bis 4 Kohlenstoffatomen; oder durch eine Trifluormethylgruppe;
ein Hydroxyradikal;
eine geradkettige oder verzweigte Alkoxygruppe mit 1 bis 6 Kohlenstoffatomen; oder
eine Cycloalkylalkyloxygruppe mit 4 bis 7 Kohlenstoffatomen
unter Ausschluß des 3-(4-Hydroxyphenyl)-5-(1-hydroxyethyl)-2-oxazolidinons, zur Herstellung eines die Monoaminoxidase, insbesondere vom Typ B, inhibierenden Mittels.